(19) Europäisches Patentamt
European Patent Office
Office européen des brevets

(11) **EP 2 510 127 B1**

(12) **EUROPEAN PATENT SPECIFICATION**

(45) Date of publication and mention
of the grant of the patent:
**10.06.2015 Bulletin 2015/24**

(21) Application number: **10836568.5**

(22) Date of filing: **07.12.2010**

(51) Int Cl.:
*C12Q 1/68* (2006.01)          *G01N 33/50* (2006.01)
*C12N 15/10* (2006.01)         *C40B 40/10* (2006.01)
*C40B 40/08* (2006.01)         *B01J 19/00* (2006.01)
*C40B 50/14* (2006.01)

(86) International application number:
**PCT/US2010/059327**

(87) International publication number:
**WO 2011/071943 (16.06.2011 Gazette 2011/24)**

(54) **PEPTIDE DISPLAY ARRAYS**

PEPTIDANZEIGEARRAYS

MATRICES DE PRÉSENTATION DE PEPTIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.12.2009 US 267425 P**

(43) Date of publication of application:
**17.10.2012 Bulletin 2012/42**

(73) Proprietor: **Prognosys Biosciences, Inc.**
**La Jolla, CA 92037 (US)**

(72) Inventors:
• **CHEE, Mark, S.**
**La Jolla, CA 92037 (US)**
• **KOZLOV, Igor, A.**
**La Jolla, CA 92037 (US)**

(74) Representative: **Green, Katherine et al**
**Mewburn Ellis LLP**
**33 Gutter Lane**
**London EC2V 8AS (GB)**

(56) References cited:
EP-A2- 1 712 623        WO-A2-03/010176
US-A1- 2003 087 232     US-A1- 2005 164 292
US-A1- 2005 260 653     US-A1- 2006 275 799
US-A1- 2008 312 103

• NIEMEYER.: 'The developments of semisynthetic
DNA?protein conjugates.' TRENDS
BIOTECHNOL vol. 20, no. 9, September 2002,
pages 395 - 401
• HENDERSON ET AL.: 'High sensitivity
multianalyte immunoassay using covalent DNA-
labeled antibodies and polymerase chain
reaction.' NUCL ACIDS RES vol. 23, no. 3, 11
February 1995, pages 522 - 529
• RAMACHANDRAN ET AL.: 'Next-generation
high-density self-assembling functional protein
arrays.' NAT METHODS vol. 5, no. 6, June 2008,
pages 535 - 538
• CHANDRA ET AL.: 'Cell-free synthesis-based
protein microarrays and their applications.'
PROTEOMICS vol. 10, no. 4, 01 December 2009,
pages 717 - 730

## Description

## FIELD OF THE INVENTION

This invention relates to peptide-based arrays, methods of producing such arrays, and related methods of use.

## BACKGROUND OF THE INVENTION

In the following discussion certain articles and methods will be described for background and introductory purposes. Nothing contained herein is to be construed as an "admission" of prior art. Applicant expressly reserves the right to demonstrate, where appropriate, that the articles and methods referenced herein do not constitute prior art under the applicable statutory provisions.

Proteomics, the study of function, structure and interaction of proteins, requires the ability to produce proteins in sufficient quantity and study these proteins in a high throughput manner. Protein microarrays are a very useful tool for such high throughput analysis of proteins, but the availability of microarray technology for large scale proteomics studies is still very limited due to the difficulty and cost of protein production (Henderson G and Bradley M, Curr Opin Biotechnol. Aug;18(4):326-30, (2007), Epub 2007 Aug 6; Tapia VE Methods Mol Biol. 2009;570:3-17 (2009)). Traditionally, peptide arrays are made by spotting pre-synthesized peptides on a surface (Salisbury CM et al, J Am Chem Soc. 2002 Dec 18;124(50):14868-70 (2002)) or by synthesizing peptides in spots on cellulose filter sheets using standard solid phase peptide synthesis, also known as the SPOT method (Frank R, J Immunol Methods. 2002 Sep 1;267(1):13-26 (2002)). The cost of generating arrays with tens to hundreds of thousands of peptides is very high, making large-scale, high throughput uses of such arrays cost prohibitive. Recently, methods for peptide array fabrication by *in vitro* translation have been developed, including protein *in situ* array (PISA) production (He M and Taussig MJ, Nucleic Acids Res, 29, e73 (2001)), nucleic acid programmable protein array (NAPPA) production (Ranachandran N et al., Science 305:86-90 (2004)), DNA to protein array (DAPA) construction (HeM Nat. Methods 5:175- 177 (2008), and arraying of proteins using *in situ* puromycin capture (Tao S-C and Zhu H, Nat. Biotech 24:1253-1254 (2006)). These approaches require individually synthesized nucleic acid templates, however, and the cost is higher than the cost of individual peptides arrayed by traditional methods.

WO 03/010176 relates to methods for the production of recombinant proteins that self-assemble into complexes with nucleic acids in solution or on supports, and protein-nucleic acid complexes produced according to the method e.g. in array format. US 2003/0087232 relates to methods for the creation and screening of polypeptides, involving partnering each protein with a unique DNA oligonucleotide tag that directs the protein to a unique site on a microarray due to specific hybridization with a complementary tag-probe on the array.

The ability to manufacture dense, high-quality, sequence-diverse peptide arrays would enable high-throughput binding and enzymatic activity profiling studies, which would have various applications in research, diagnostics and therapeutic development. The present invention addresses this need.

## SUMMARY OF THE INVENTION

This Summary is provided to introduce a selection of concepts in a simplified form that are further described below in the Detailed Description. This Summary is not intended to identify key or essential features of the claimed subject matter, nor is it intended to be used to limit the scope of the claimed subject matter. Other features, details, utilities, and advantages of the claimed subject matter will be apparent from the following written Detailed Description including those aspects illustrated in the accompanying drawings and defined in the appended claims.

The present invention provides arrays, methods of constructing arrays, and methods of use of such arrays, as set out in the claims.

The arrays of the invention comprise a substrate with two or more discrete constructs or discrete sets of constructs associated on the surface of the substrate, optionally via a linker molecule. The constructs comprise both an oligonucleotide encoding a peptide of interest and the peptide of interest itself. The oligonucleotide is associated with the substrate surface, and the peptide is associated with the oligonucleotide, either directly or via a linker molecule.

In specific aspects, the constructs of the array include a first oligonucleotide region encoding a peptide of interest and an affinity tag, a second oligonucleotide region positioned 5' to the first oligonucleotide comprising an untranslated region, a fusion peptide comprising both the peptide of interest and the affinity tag; and a capture agent associated with the oligonucleotide that forms a binding pair with the affinity tag of the fusion protein. The untranslated region of the oligonucleotide comprises a transcriptional start site and a ribosomal binding site 5' to the region encoding the peptide of interest and the affinity tag.

The capture agent may be associated with the oligonucleotide directly or via a linker molecule. In a preferred aspect, the fusion peptide is associated with the oligonucleotide at the terminus of the oligonucleotide distal to the substrate surface via binding to a capture agent associated directly or indirectly with the oligonucleotide. In certain other aspects, the peptide is associated with the oligonucleotide near the surface of the substrate, either via a capture agent associated with the oligonucleotide or a capture agent associated with the linker to which the oligonucleotide attaches to the substrate surface. In a preferred aspect, the capture agent is associated at a position on the oligonucleotide distal to the substrate surface via a primer sequence linked to the oligonucleotide, e.g., using a synthetic linker molecule.

In certain aspects, the array substrate is a planar support, a film, beads or a combination thereof. One strand of the oligonucleotide comprises a primer region used for introduction of an untranslated region to the oligonucleotide. The untranslated region comprises a ribosomal start site and a transcriptional promoter region for the initiation of the transcription reaction.

In one particular aspect, the constructs are arrayed on the surface of a flow cell, and preferably a flow cell used for high throughput sequencing. The sequences can optionally be clonally amplified *in situ* in the flow cell. In this aspect, the sequences are arrayed randomly, but the identity of each clonal sequence can be determined either before or after the production of protein and its use in a screening assay. The ability to array many millions of DNA templates and determine their sequence can be done quite inexpensively, e.g., by randomization of bases at defined positions during synthesis of an oligo template, by combining shorter oligos to form a longer template, or by deriving a library of sequences from a genomic DNA or cDNA library.

In some aspects of the invention, the constructs of the arrays comprise double-stranded oligonucleotides associated with the peptide. In other aspects, it may be desirable to remove one of the oligonucleotide strands following peptide association, and the arrays will comprise a single-stranded oligonucleotide associated with the peptide of interest.

The invention also provides methods of constructing an array of the invention. Specific methods of producing peptide arrays of the invention utilize sets of nucleic acids synthesized on a substrate surface *(e.g., a planar substrate or a set of beads)*. Nucleic acids of known sequence encoding amino acid sequences are preferably synthesized directly onto the surfaces, e.g., using chemical synthesis techniques. The identity of the sequences at each different location or site on the array may be predetermined or determined following synthesis on the solid substrate or assembly (e.g., in the case of beads) into a fixed format, such as a microarray. The sequence identities may also be determined after construction of the peptide array. Following the synthetic production of the nucleic acids, additional nucleic acids comprising desired sequence information (e.g., sequences encoding elements of the peptide array, sequences necessary for transcription or translation, and the like) are attached to the synthesized nucleic acids on the substrate surface using methods such as chemical and preferably enzymatic ligation, primer extension, amplification, etc. The extended nucleic acids are used as templates for the production of peptides of interest and other peptide elements *(e.g., affinity tags)* via *in vitro* transcription and translation.

In another specific aspect for constructing a peptide array, nucleic acid microarrays comprising a set of chemically synthesized oligonucleotides encoding amino acid sequences are manufactured. Additional nucleic acids comprising desired sequence information (e.g., sequences encoding elements of the peptide array, sequences necessary for transcription or translation, and the like) are attached to the oligonucleotides of these arrays using methods such as chemical and preferably enzymatic ligation, primer extension, amplification, etc. The extended nucleic acids are used as templates for the production of peptides of interest and other peptide elements *(e.g.,* affinity tags) via *in vitro* transcription and translation.

Following peptide production, the peptides are captured at known locations on the substrate surface. Preferably, the peptides are associated with the locations of their respective nucleic acid templates, or directly with the actual templates, using the methods described herein in more detail. These methods of the invention utilizing a combination of synthetic and post-synthetic nucleic acid construction are particularly useful in the creation of large numbers of peptides of known sequence on a substrate surface. For example, using these methods, arrays of at least 100, at least 1000, at least 5000 and at least 10,000 resolvable peptides can be produced on a substrate surface.

Alternatively, the extended nucleic acid sequences are transferred (either directly or as copies) to the surface and amplified in situ, where the rest of the process of peptide array construction is carried out.

In some aspects, the arrays can be produced using a surface comprising two or more constructs comprising a single-stranded oligonucleotide template region encoding a peptide of interest and an affinity tag. In one aspect, single-stranded oligonucleotide templates are converted into a double-stranded oligonucleotide region, and an untranslated region comprising a transcription start site and a ribosomal binding site is introduced to the 5' end of the double-stranded oligonucleotide region. In other preferred aspects, the untranslated region is added to the single-stranded region prior to conversion of the single-stranded oligonucleotide to a double-stranded template. The double-stranded oligonucleotide region is then subjected to an *in vitro* transcription and translation event to produce a fusion peptide comprising the peptide of interest and the affinity tag, and the fusion peptide is captured in the construct by binding of the affinity tag portion of the fusion peptide to a capture agent associated directly or indirectly with the oligonucleotide. The capture agent is optionally associated with the untranslated region of the oligonucleotide and is introduced to the oligonucleotide via a region complementary to a region of this untranslated area. Alternatively, the capture agent may be associated with the construct via the peptide coding region or via a linker attaching the oligonucleotide template to the substrate surface.

In another aspect, the arrays can be produced using a surface comprising two or more constructs comprising a single-stranded oligonucleotide template region comprising a sequence complementary to a sequence encoding a peptide of interest and complementary to a sequence encoding an affinity tag. The single-stranded oligonucleotide templates are converted into a double-stranded oligonucleotide region, and an untranslated region comprising a transcription start site and a ribosomal binding site is introduced to the 5' end of the double-stranded oligonucleotide region. The double-stranded oligonucleotide region is then subjected to a transcription and translation event to produce a peptide comprising the peptide of interest and the affinity tag, and the fusion peptide is captured in the construct by binding of the affinity tag of the fusion peptide to a capture agent. The capture agent optionally is associated with the untranslated region and introduced to the construct with this region. Alternatively, the capture agent may be associated with the construct via the peptide encoding region or via a linker attaching the oligonucleotide to the substrate surface.

In yet another aspect, the invention provides a method of constructing an array by providing a substrate comprising a surface with two or more constructs comprising a single-stranded oligonucleotide encoding a peptide of interest and an affinity tag. A second, universal oligonucleotide comprising an untranslated region with a transcriptional start site and a ribosomal binding site is attached to the oligonucleotides of the construct, and a capture agent is associated with the oligonucleotides via hybridization to the universal oligonucleotide. The single-stranded oligonucleotide regions of the constructs are converted into double-stranded oligonucleotide regions; and the double-stranded oligonucleotide template region is subjected to a transcription and translation event to produce a fusion peptide comprising the peptide of interest and the affinity tag. The fusion protein produced from this translation event is captured by the capture agent via the peptide's affinity tag. The resulting array comprises discrete units comprising a nucleic acid associated with the peptide encoded by the nucleic acid. These discrete construct units can comprise one or more constructs that are distinguishable from other constructs having separate peptides of interest.

In yet another aspect, the invention provides a method of constructing an array by providing a substrate comprising a surface with two or more constructs comprising a single-stranded oligonucleotide encoding a peptide of interest and an affinity tag. A second, universal oligonucleotide comprising an untranslated region with a transcriptional start site and a ribosomal binding site is attached to the oligonucleotides of the construct, and a capture agent is associated with the oligonucleotides via hybridization to the universal oligonucleotide. The single-stranded oligonucleotide regions of the constructs are converted into double-stranded oligonucleotide regions; and the double-stranded oligonucleotide template region is subjected to a transcription and translation event to produce a fusion peptide comprising the peptide of interest and the affinity tag. The fusion protein produced from this translation event is captured by the capture agent via the peptide's affinity tag. The resulting array comprises discrete units comprising a nucleic acid associated with the peptide encoded by the nucleic acid. These discrete construct units can comprise one or more constructs that are distinguishable from other constructs having separate peptides of interest.

In yet another aspect, the invention provides a method of constructing an array by providing a substrate comprising a surface with two or more constructs comprising a single-stranded oligonucleotide comprising a region encoding a peptide of interest and an affinity tag and an untranslated region, where the oligonucleotide is attached to the substrate surface at its 5' end. Primer annealing to the untranslated region of the oligonucleotide creates a double stranded region at the transcriptional starts site, which is efficient to initiate transcription and translation of the peptide encoding the peptide of interest and the affinity tag. The constructs of the array are not converted into a double-stranded oligonucleotide prior to the initiation of transcription and translation.

The invention also provides methods for detecting binding of an agent to a peptide of interest, including detection of the presence or absence of an agent in a sample. These methods comprise providing an array comprising: a substrate having a surface; two or more individual constructs associated on the surface of the substrate, the individual constructs having both an oligonucleotide encoding a peptide of interest and the peptide of interest itself. In specific aspects, the arrays used in these detection methods have two or more constructs comprising: an oligonucleotide region encoding a peptide of interest, an oligonucleotide region encoding an affinity tag, and an untranslated region; a peptide comprising the peptide of interest and an affinity tag; and a capture agent that selectively binds to the affinity tag. Detection of the binding of an agent to the peptide of interest is accomplished by exposing the array to an agent and detecting the presence or absence of binding of the agent to the peptide of interest. The individual constructs are associated with the array through the association of the oligonucleotide with the substrate surface, and the peptide is associated with the oligonucleotide e.g., through binding of the affinity tag to the capture agent.

## DESCRIPTION OF THE FIGURES

FIGs. 1A-1F illustrates exemplary constructs that are used in the composite oligonucleotide-peptide arrays of the invention.
FIG. 2 illustrates an array comprising sets of identical constructs comprising the same fusion peptides.
FIG. 3 illustrates an array comprising sets of constructs comprising the same oligonucleotide region in different configurations.

FIG. 4 illustrates an array comprising constructs with the same peptide but with different affinity tags and capture agents.

FIG. 5 illustrates a first general approach for producing the composite oligonucleotide-peptide arrays of the invention.

FIG. 6 illustrates a second general approach for producing the composite oligonucleotide-peptide arrays of the invention using capture agents.

FIG. 7 illustrates a first production method using capture agents and oligonucleotide templates on beads.

FIG. 8 illustrates a second production method using capture agents and oligonucleotide templates on a planar surface.

FIG. 9 illustrates a third production method using puromycin capture of the peptide produced by the oligonucleotide template.

FIG. 10 illustrates a more specific aspect of the production method of FIG. 9 using post translational modification.

FIG. 11 illustrates the LCTPSR peptide using FGE using the method illustrated in FIG. 10.

FIG. 12 illustrates a general assay system utilizing a sequencing flow cell.

FIG. 13 illustrates the use of various construct configurations on an array of the invention.

FIG. 14 illustrates the use of diffusion to create a halo effect to reduce false positives on an array of the invention.

FIG. 15 illustrates the peptide binding results obtained using the bead arrays of FIG. 7.

FIG. 16 illustrates the peptide binding results obtained using the slide arrays produced as shown in FIG. 8.

## DEFINITIONS

The terms used herein are intended to have the plain and ordinary meaning as understood by those of ordinary skill in the art. The following definitions are intended to aid the reader in understanding the present invention, but are not intended to vary or otherwise limit the meaning of such terms unless specifically indicated.

The term "affinity tag" as used herein refers to one member of a binding pair that selectively binds to a capture agent.

The term "binding pair" means any two molecules that are known to selectively bind to one another. In the case of two proteins, the molecules selectively bind to one another as described in more detail herein. Such binding may include covalent and/or non-covalent binding. Examples include, but are not limited to, biotin and avidin; biotin and streptavidin; an antibody and its particular epitope; and the like.

The term "capture agent" as used herein refers any moiety that allows capture of a peptide via binding to or linkage with an affinity tag of the peptide. The binding between the capture agent and its affinity tag may be a covalent bond and/or a non-covalent bond. A capture agent includes, e.g., a member of a binding pair that selectively binds to an affinity tag on a fusion peptide, a chemical linkage that is added by recombinant technology or other mechanisms, and the like. In a particular aspect, the capture agent is an antibody that selectively binds to an affinity tag epitope. Capture agents can be associated with a construct using conventional techniques including hybridization, crosslinking (e.g., covalent immobilization using psoralen), introduction through post-translational modification and the like.

The term "complementary" refers to the topological compatibility or interactive structure of interacting surfaces of a nucleic acid binding pair. Preferred complementary structures have binding affinity for each other and the greater the degree of complementarity the nucleic acids have for each other the greater the hybridization between the structures.

The term "diagnostic tool" as used herein refers to any composition or assay of the invention used in order to carry out a diagnostic test or assay on a patient sample. As a diagnostic tool, the composition of the invention may be considered a collection of analyte specific reagents, and as such may form part of a diagnostic test regulated by a federal or state agency. The use of the compositions of the invention as a diagnostic tool is not intended to be related to any use of the composition in the development of therapeutic agents.

The term "oligonucleotide" is used herein to mean a linear polymer of nucleotide monomers. As used herein, the term may refer to single stranded or double stranded forms. Monomers making up nucleic acids and oligonucleotides are capable of specifically binding to a natural polynucleotide by way of a regular pattern of monomer-to-monomer interactions, such as Watson-Crick type of base pairing, base stacking, Hoogsteen or reverse Hoogsteen types of base pairing, or the like, to form duplex or triplex forms. Such monomers and their internucleosidic linkages may be naturally occurring or may be analogs thereof, e.g., naturally occurring or non-naturally occurring analogs. Non-naturally occurring analogs may include peptide nucleic acids, locked nucleic acids, phosphorothioate internucleosidic linkages, bases containing linking groups permitting the attachment of labels, such as fluorophores, or haptens, and the like. Whenever the use of an oligonucleotide or nucleic acid requires enzymatic processing, such as extension by a polymerase, ligation by a ligase, or the like, one of ordinary skill would understand that oligonucleotides or nucleic acids in those instances would not contain certain analogs of internucleosidic linkages, sugar moieties, or bases at any or some positions, when such analogs are incompatible with enzymatic reactions. Nucleic acids typically range in size from a few monomeric units, *e.g.,* 5-40, when they are usually referred to as "oligonucleotides," to several hundred thousand or more monomeric units. Whenever a nucleic acid or oligonucleotide is represented by a sequence of letters (upper or lower case), such as "ATGCCTG," it will be understood that the nucleotides are in 5'>3' order from left to right and that "A" denotes deoxyadenosine, "C" denotes deoxycytidine, "G" denotes deoxyguanosine, and "T" denotes deoxythymidine, "I" denotes deoxyinosine, "U" denotes uridine, unless otherwise indicated or obvious from context. Usually nucleic acids comprise the natural nucleosides (e.g., deoxyadenosine, deoxycytidine, deoxyguanosine, deoxythymidine for DNA or their ribose counterparts for RNA) linked by phosphodiester linkages; however, they may also comprise non-natural nucleotide analogs, e.g., modified bases, sugars, or internucleosidic linkages. To those skilled in the art, where an enzyme has specific oligonucleotide or nucleic acid substrate requirements for activity, e.g., single-stranded DNA, RNA/DNA duplex, or the like, then selection of appropriate composition for the oligonucleotide or nucleic acid substrates is well within the knowledge of one of ordinary skill, especially with guidance from treatises, such as Sambrook et al., Molecular Cloning, Second Edition (Cold Spring Harbor Laboratory, New York, 1989), and like references.

The terms "peptide", "polypeptide," and the like are used interchangeably herein, and refer to a polymeric form of amino acids of any length, which can include coded and non-coded amino acids, chemically or biochemically modified or derivatized amino acids, and polypeptides having modified peptide backbones.

The term "research tool" as used herein refers to any composition or assay of the invention used for scientific enquiry, academic or commercial in nature, including the development of pharmaceutical and/or biological therapeutics. The research tools of the invention are not intended to be therapeutic or to be subject to regulatory approval; rather, the research tools of the invention are intended to facilitate research and aid in such development activities, including any activities performed with the intention to produce information to support a regulatory submission.

The term "selectively binds", "selective binding" and the like as used herein, when referring to a binding partner (e.g., protein, nucleic acid, antibody, etc.), refers to a binding reaction of two or more binding partners with high affinity and/or complementarity to ensure selective hybridization under designated assay conditions. Typically, specific binding will be at least three times background signal or noise and more typically more than 10 to 100 times background. Thus, under designated conditions the binding partner binds to its particular "target" molecule and does not bind in a significant amount to other molecules present in the sample.

The term "$T_m$" is used in reference to the "melting temperature." The melting temperature is the temperature at which a population of double-stranded nucleic acid molecules becomes half dissociated into single strands. Several equations for calculating the $T_m$ of nucleic acids are well known in the art. As indicated by standard references, a simple estimate of the $T_m$ value may be calculated by the equation, $T_m = 81.5 + 0.41$ (% G+C), when a nucleic acid is in aqueous solution at 1M NaCl (see e.g., Anderson and Young, Quantitative Filter Hybridization, in Nucleic Acid Hybridization (1985)). Other references (e.g., Allawi, H. T. & SantaLucia, J., Jr., Biochemistry 36, 10581-94 (1997)) include alternative methods of computation which take structural and environmental, as well as sequence characteristics into account for the calculation of $T_m$.

## DETAILED DESCRIPTION OF THE INVENTION

The practice of the techniques described herein may employ, unless otherwise indicated, conventional techniques and descriptions of organic chemistry, polymer technology, molecular biology (including recombinant techniques), cell biology, biochemistry, and sequencing technology, which are within the skill of those who practice in the art. Such conventional techniques include polymer array synthesis, hybridization and ligation of polynucleotides, and detection of hybridization using a label. Specific illustrations of suitable techniques can be had by reference to the examples herein. However, other equivalent conventional procedures can, of course, also be used. Such conventional techniques and descriptions can be found in standard laboratory manuals such as Green, et al., Eds. (1999), Genome Analysis: A Laboratory Manual Series (Vols. I-IV); Weiner, Gabriel, Stephens, Eds. (2007), Genetic Variation: A Laboratory Manual; Dieffenbach, Dveksler, Eds. (2003), PCR Primer: A Laboratory Manual; Bowtell and Sambrook (2003), DNA Microarrays: A Molecular Cloning Manual; Mount (2004), Bioinformatics: Sequence and Genome Analysis; Sambrook and Russell (2006), Condensed Protocols from Molecular Cloning: A Laboratory Manual; and Sambrook and Russell (2002), Molecular Cloning: A Laboratory Manual (all from Cold Spring Harbor Laboratory Press); Stryer, L. (1995) Biochemistry (4th Ed.) W.H. Freeman, New York N.Y.; Gait, "Oligonucleotide Synthesis: A Practical Approach" 1984, IRL Press, London; Nelson and Cox (2000), Lehninger, Principles of Biochemistry 3rd Ed., W. H. Freeman Pub., New York, N.Y.; and Berg et al. (2002) Biochemistry, 5th Ed., W.H. Freeman Pub., New York, N.Y.

Note that as used herein and in the appended claims, the singular forms "a," "an," and "the" include plural referents unless the context clearly dictates otherwise. Thus, for example, reference to "an array" refers to one or more such arrays, and reference to "the method" includes reference to equivalent steps and methods known to those skilled in the art, and so forth.

Unless defined otherwise, all technical and scientific terms used herein have the same meaning as commonly understood by one of ordinary skill in the art to which this invention belongs.

Where a range of values is provided, it is understood that each intervening value, between the upper and lower limit of that range and any other stated or intervening value in that stated range is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either both of those included limits are also included in the invention.

In the following description, numerous specific details are set forth to provide a more thorough understanding of the present invention. However, it will be apparent to one of skill in the art that the present invention may be practiced without one or more of these specific details. In other instances, well-known features and procedures well known to those skilled in the art have not been described in order to avoid obscuring the invention.

*The Invention in General*

The arrays of the present invention provide novel array compositions and methods of producing these arrays in a cost-effective manner. The arrays of the invention comprise both an oligonucleotide peptide-coding template, and preferably a double-stranded oligonucleotide peptide-coding template, associated with a peptide encoded by the oligonucleotide template. The microarrays of the invention are produced using an *in vitro* transcription/translation of oligonucleotide templates obtained by very inexpensive microarray based synthesis. The oligonucleotides of the array are converted into oligopeptide composite constructs by transcribing the oligonucleotides attached to the array surface into RNA templates followed by translation of these templates into peptides. The translated peptide is then associated to the oligonucleotide template via different mechanisms, including the use of a chemical linker group, the use of a capture agent, and the like. The variety of peptides available on the array reflects the complexity of the original oligonucleotide templates on the array.

In a particular aspect of the invention, the arrays comprise a capture agent that is used to bind to an affinity tag region on the peptide. In this instance, the peptide is a fusion peptide comprising an affinity tag region which binds the capture agent at the C-terminal region, and the peptide(s) of interest at the N-terminal region. The affinity tag is encoded by the oligonucleotide template in the construct, and is used to capture the fusion peptide created through transcription and translation.

In a preferred aspect, multiple peptides on the array will comprise the same affinity tag, allowing the use of a common capture agent to be used in the constructs. For example, an array may comprise peptides of varying sequence having a single common affinity tag and capture agent. This allows the use of a single peptide binding moiety to be added during the construction of the array, significantly decreasing the complexity and cost of production.

In another example, the array may comprise two or more constructs having the same peptide sequence of interest, but with different affinity tags and capture agents. This latter approach allows formation of arrays having internal control mechanisms to ensure that the activity of the peptide of interest is not adversely affected by the process of constructing or using the array.

The methods of protein microarray production of the instant invention are reliable and reproducible, and capable of producing identical arrays of the same quality and protein quantity. The arrays of the invention are useful in the display of a wide variety of peptides, including proteins and fragments thereof from various classes and sizes. The yield of protein per array unit is sufficiently high to allow reproducible and discrete detection and/or activity for the particular array units. This allows the arrays to be produced on a large scale with extremely high density at a relatively low cost.

Importantly, the arrays of the invention have the ability to display functional peptides, including entire proteins, peptide domains, active sites of proteins, and the like. It is a feature of the invention that the peptide arrays can be created to study functional proteins that are generally difficult to isolate from *in vivo* sources, *e.g.,* insoluble proteins such as prions or beta amyloid peptides.

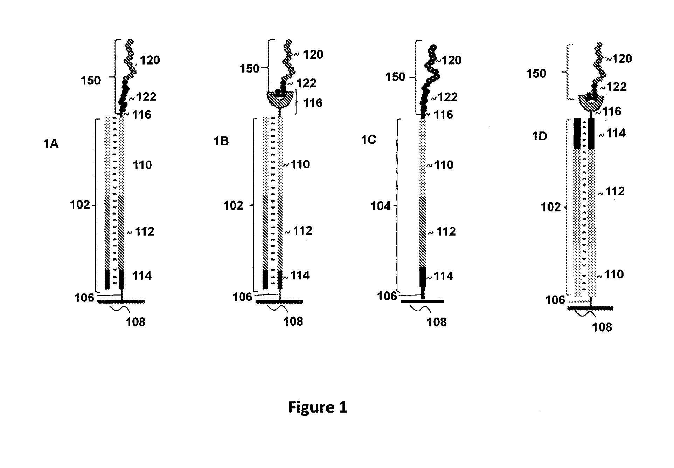

The arrays of the invention comprise double-stranded or single-stranded oligonucleotides associated with both a substrate surface and a peptide of interest. Three exemplary molecules are illustrated in FIGs. 1A through 1F. In FIG. 1A, the array construct comprises an oligonucleotide portion 102 comprising an untranslated region 110, a region 112 encoding a peptide of interest, and a region 114 encoding an affinity tag and a stop codon. The oligonucleotide may be directly associated with the surface, or optionally attached to the array using a linker 106. Generally, a single strand is attached to the array surface, and the strand may be attached at either its 3'or 5' end. The portion of the oligonucleotide distal to the array comprises a peptide association moiety 116 which allows the peptide produced from the oligonucleotide template to be associated with the construct following *in vitro* transcription and translation. The associated peptide 150 comprises the peptide of interest 120 which is encoded by the coding region 112 of the oligonucleotide, fused to the affinity tag 122 which is encoded by region 114 of the oligonucleotide. The affinity tag 122 allows attachment of the peptide 150 to the oligonucleotide. In FIG. 1B, the peptide association moiety 116 comprises a capture agent, *e.g.,* an antibody or a member of a protein binding pair. In FIG. 1C, the oligonucleotide construct may have a strand removed following transcription and translation, resulting in a single-stranded molecule 104 with either a sequence encoding the peptide or a sequence complementary to the sequence encoding the peptide. This single strand may be attached to the substrate surface at either its 3'or 5' end.

Although the constructs are illustrated with the untranslated region proximal to the capture agent, the constructs may be configured so that the untranslated region is attached to the substrate surface and the region encoding the affinity tag is proximal to the capture agent. For example, FIG. 1D illustrates a construct having the same components as those in FIG. 1B, but in which the orientation of the oligonucleotide 102 is effectively flipped.

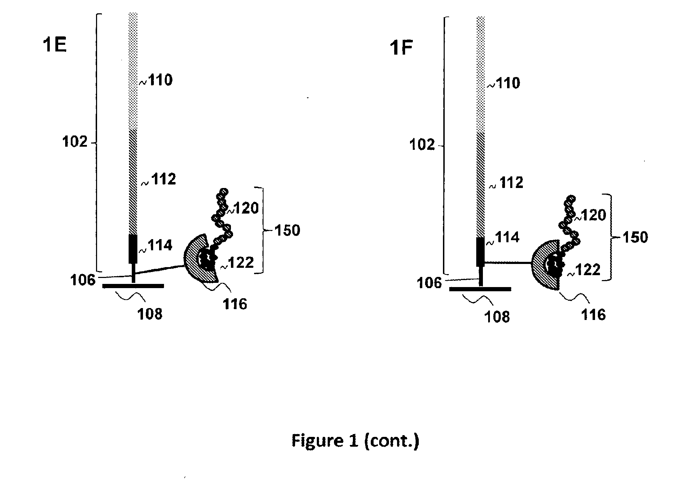

In FIGs. 1E and 1F, the capture agent 116 is associated proximal to the substrate surface relative to the oligonucleotide. The capture agent 116 can be associated with the construct via either the linker 106 that attaches the oligonucleotide portion to the substrate surface 108 (as illustrated in 1E) or via the oligonucleotide 102 itself (as illustrated in IF). The oligonucleotide constructs 102 shown here may be either single-stranded as illustrated in FIG. 1C, or double-stranded, as illustrated in FIGs. 1A, 1B and 1D. Generally, a single strand is attached to the array surface, and the strand may be attached to the substrate surface at either its 3'or 5' end.

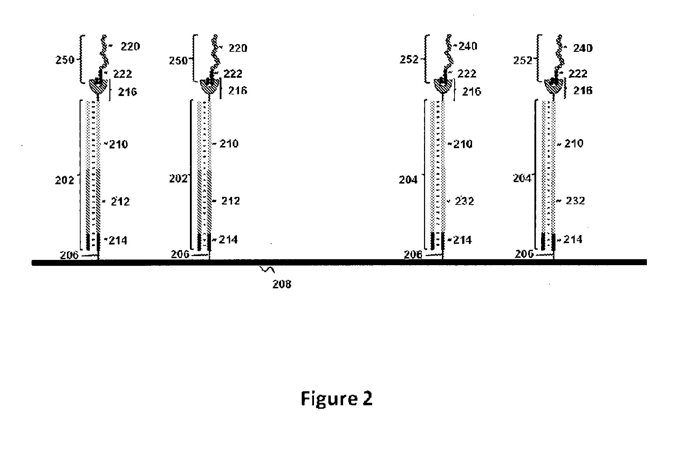

The arrays may contain individual constructs that themselves are discrete, interrogatable units for analysis of the peptide of interest. For certain applications, however, it is desirable to increase the intensity of signal created on the array. The invention thus also includes arrays having discrete interrogatable units comprising two or more identical constructs that display the same peptide of interest. For example, in FIG. 2, the array comprises a substrate having different constructs positioned on the array in discrete groups of two or more constructs, which preferably utilize the same capture agents and affinity tags for association of the peptides to the oligonucleotide. The constructs of the first set comprise an oligonucleotide portion 202 comprising an untranslated region 210, a region 212 encoding a first peptide of interest, and a region 214 encoding an affinity tag and a stop codon. The constructs of the second set comprise an oligonucleotide portion 204 comprising an untranslated region 210, a region 232 encoding a second peptide of interest, and a region 214 encoding an affinity tag and a stop codon. The oligonucleotides of both sets may be directly associated with the surface, or optionally attached to the array using linkers 206. The portions of the oligonucleotide distal to the array comprises peptide association moieties 216 which allows the peptides produced from the oligonucleotide templates to be associated with the construct following *in vitro* transcription and translation. The associated peptides 250 of the first set comprise the first peptide of interest 220 fused to the affinity tag 222 which is encoded by 214. The associated peptides of the second set 252 comprise the second peptide of interest 240 fused to the same affinity tag 222 as the first set, which as in the first construct is encoded by a common region 214.

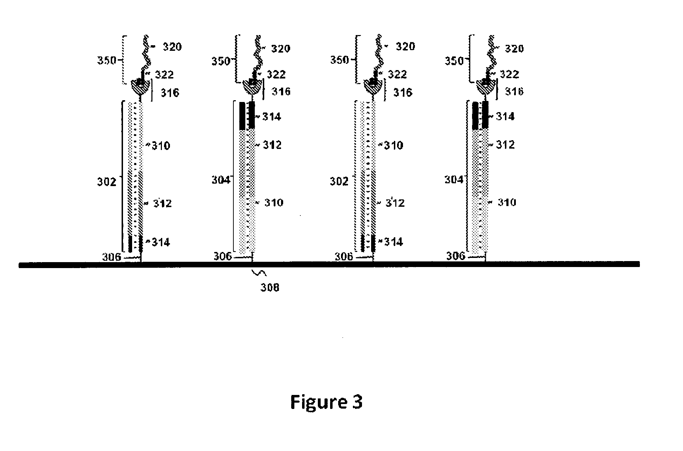

In other aspects, the array may comprise individual constructs that have the same oligonucleotide regions in a different relationship with respect to the substrate. In FIG. 3, the constructs have the same components as the constructs of FIGs. 1B and 1D, with one set of constructs having at least one construct on the array in the configuration of FIG. 1B and a second set of constructs having at least one construct on the array in the configuration of FIG. 1D. The constructs of the first set comprise an oligonucleotide portion 302 comprising an untranslated region 310, a region 312 encoding a first peptide of interest, and a region 314 encoding an affinity tag and a stop codon, with the latter region 314 being attached to the substrate surface. The constructs of the second set comprise an oligonucleotide portion 304 comprising the untranslated region 310, region 312 encoding a first peptide of interest, and region 314 encoding an affinity tag and a stop codon, but in this configuration the first region 310 is attached to the substrate surface. The oligonucleotides of both sets may be directly associated with the substrate surface, or optionally attached to the array using linkers 306, and they may be attached to the surface via either the 3' or the 5' end. The portions of the oligonucleotides distal to the substrate surface comprise peptide association moieties 316 which allow the peptides produced from the oligonucleotide templates to be associated with the construct following *in vitro* transcription and translation.

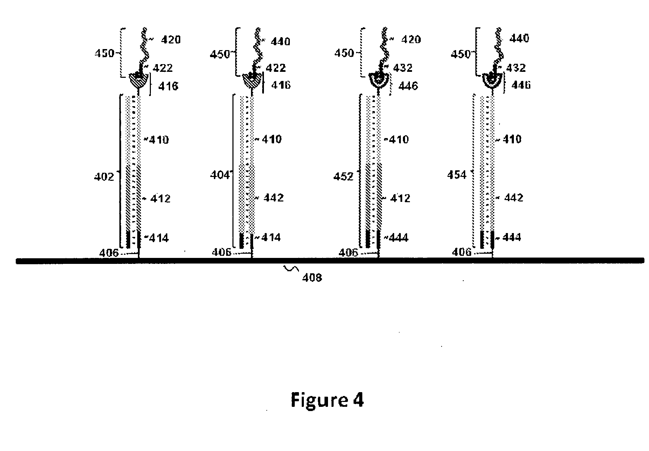

In other aspects of the invention, it is desirable to have two or more constructs on the array comprising the same peptide of interest fused to different affinity tags and associated with different capture agents on the oligonucleotide. This is illustrated in FIG. 4. Here, the illustrated array comprises individual constructs comprising oligonucleotides that encode the same peptides of interest, but where each peptide is associated with two different capture agents on the oligonucleotide. A first set of oligonucleotides 402 and 452 comprise the same untranslated region 410 and region 412 encoding a first peptide of interest 420, but they are linked to different regions 414 and 444 encoding different affinity tags. The peptides produced from these two constructs will comprise the same peptide of interest, but fused to two different affinity tags that are captured using two different capture agents 416, 446. Similarly, a second set of oligonucleotides 404 and 454 comprise the same untranslated region 410 and region 442 encoding a second peptide of interest 440, but they are linked to different regions 414 and 444 encoding different affinity tags 422, 432.

General methods for the process of peptide array fabrication

The arrays of the invention can be produced using initial made-to-order oligonucleotide arrays using any of a number of technologies. The arrays can be produced on a planar surface, or on a series of discrete surfaces, e.g. beads, that together form an array. The composite oligo-peptide arrays can be produced using any single-stranded or double-stranded oligonucleotide array.

Suitable methods for such oligonucleotide array production can be found, for example, in US Pat. No. 7,556,919, issued July 2009 to Chenchik et al.; US Pat.

No. 7,291,471 issued November 2007 to Sampson et al.; US Pat. No. 6,294,336, issued September 2001 to Boyce-Jacino et al.; US Pat. No. 6,291,183, issued September 2001 to Pirrung et al.; US Pat. No. 6,284,497 issued September 2001 to Sabanayagam et al.; US Pat. No. 6,261,776 issued July 2001 to Pirrung et al.; US Pat. No. 6,222,030 issued April 2001 to Dellinger et al.; US Pat. No. 6,087,112, issued July 2000 to Dale; 6,077,674 issued June 200 to Schliefer and May; US Pat. No. 5,919,523 issued July 1999 to Sundberg et al.; US Pat. No. 5,861,242 issued January 1999 to Chee et al.; US Pat. No. 5,856,174 issued January 1999 to Lipshutz et al; US Pat. No. 5,837,832 issued Nov 1998 to Chee et al.; US Pat. No. 5,770722, issued June 19989 to Lockhart et al; US Pat. No. 5,750,669, issued May 1998 to Rosch et al; US Pat. No. 5,843,655 issued December 1998 to McGall; US Pat. No. 5,700,637, issued Dec 1997 to Southern; US Pat. No. 5,723,320 issued March 1998 to Dehlinger; US Pat. No. 5,695,940 issued Dec 1997 to Drmanac et al.; US Pat. No. 5,631,724 issued May 1997 to Stern et al.; US Pat. No. 5,556,752 issued Sept 1996 to Lockhart et al.; US Pat. No. 5,525,464 issued July 1996 to Drmanac et al; US Pat. No. 5,492,806 issued February 1996 to Drmanac et al; US Pat. No. 5,445,934 issued August 1995 to Fodor et al.; and US Pat. No. 5,436,327 issued July 1995.

Using these or other such array technologies, many oligonucleotide templates can be synthesized in parallel on an array. In addition to encoding for the peptide of interest (or a sequence complementary to that encoding a peptide of interest), the single-stranded oligonucleotides comprise universal primer sequences corresponding to an untranslated region or a region complementary to a universal untranslated region. In addition, the oligonucleotide is associated with a region for the capture of the peptide following translation.

The oligonucleotide can be synthesized to have each of these elements, or an oligonucleotide can be constructed on the substrate by addition of the various elements to an initial oligonucleotide associated with the substrate surface. For example, in one example, the oligonucleotides used in construction of the array comprise regions encoding an affinity tag and stop codon at one end of the oligonucleotide and a primer region used to attach a long universal untranslated region at the other end of the oligonucleotide. Where single-stranded oligonucleotides are used in construction of the array, the single-stranded oligonucleotide also comprises a region complementary to a primer that is used to synthesize a strand complementary to the single-stranded oligonucleotides, resulting in extension of the single-stranded oligonucleotide into a double-stranded oligonucleotide template for use in the *in vitro* transcription and translation reactions.

In a preferred aspect, the oligonucleotides will comprise the primer for attachment of the untranslated region at the 5' end of the oligonucleotide and the sequence encoding the affinity tag at the 3' end of the oligonucleotide. Alternatively, the substrate-bound single-stranded oligonucleotides may comprise the reverse complement sequences, and the strand complementary to the initial oligonucleotide will be transcribed. In the latter case, single-stranded oligonucleotides comprising sequences complementary to the untranslated region and the affinity tag are initially bound to the substrate.

It is a feature of the constructs on the arrays of the invention that peptide fragments or peptides having deletions will be selected against, as fusion peptides expressed from the templates that contain deletions or insertions (e.g., from byproducts of oligonucleotide synthesis or mistakes in transcription) will generally not be captured on the array because they will have a frame shift during translation and will not display the correct sequence of the affinity tag.

The untranslated region preferably comprises a transcriptional promoter region at the 5'-end followed by a ribosomal binding site (RBS) that is used to initiate the transcription and translation events to produce the peptides of the array. The untranslated sequence can be included in the initially synthesized oligonucleotide, or it can be attached to the oligonucleotide template using various techniques. In one example, the 5' primer region of the oligonucleotide may comprise a restriction endonuclease site, and the universal untranslated region can be added to the 5' of the oligonucleotide by digestion and ligation. In another example, a primer complementary to both the 5' primer region and the universal untranslated region can be used for splint ligation of the two molecules.

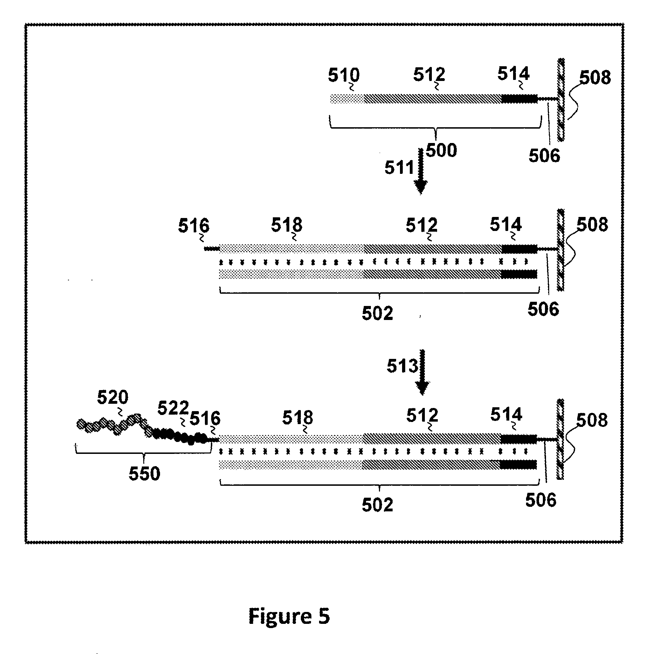

FIG. 5 illustrates a general scheme for production of the arrays of the invention using single-stranded oligonucleotide arrays. The method begins with oligonucleotides 500 arrayed on a surface 508, optionally attached to the surface via a linker sequence 506. The oligonucleotides 500 each comprise a first region 514 that encodes an affinity tag and a stop codon, and which comprises a sequence that hybridizes selectively to a primer used in primer extension of the oligonucleotide, a coding region 512 encoding a peptide or complementary to a region encoding a peptide, and a second primer region 510 for attachment of a universal untranslated region 518 and/or a capture agent 516 for the encoded peptide. The capture agent 516 may be a chemical linkage group that interacts with and binds to the affinity tag. [

Extension of the single-stranded molecule from the primer region 514 and attachment of the untranslated region in step 511 produces a double-stranded template 502 that includes the capture agent 516 on the construct positioned distally to the substrate 508. In this example, the coding region 512 encodes the peptide of interest, and the primer extension region 514 comprising a coding region for an affinity tag that binds to a capture agent 516 and a stop codon. *In vitro* transcription and translation will produce the fusion peptide 550, comprising both the peptide of interest 520 and the affinity region 522, the latter of which associates with the capture agent 516 following peptide synthesis.

In certain aspects of the invention, the DNA is created as a double-stranded molecule. To add the capture agent to this molecule, it can be tailed with terminal transferase to add a single-stranded oligo dT region to which a capture region associated with an oligo dA can hybridize.

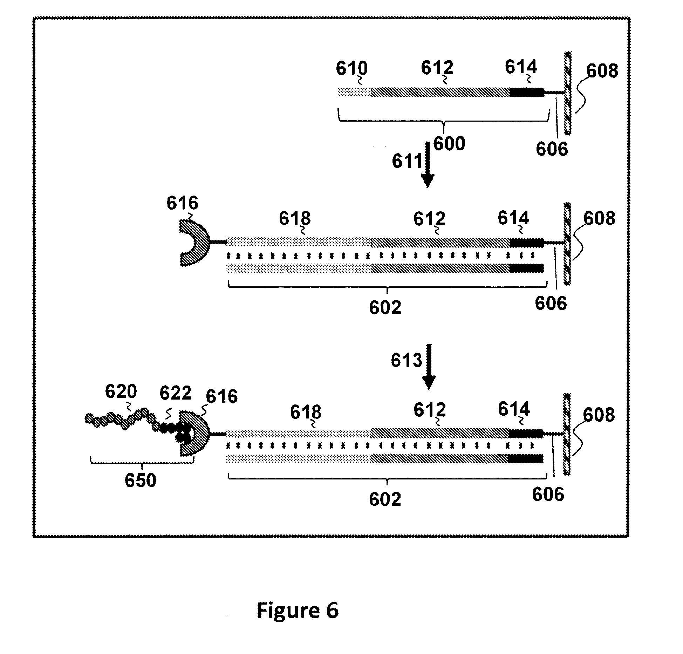

FIG. 6 illustrates another general scheme for production of the arrays of the invention using single-stranded oligonucleotide arrays and a capture agent. The method begins with oligonucleotides 600 arrayed on a surface 608, optionally attached to the surface via a linker sequence 606. The oligonucleotides 600 each comprise a first region 614 that encodes an affinity tag and a stop codon, and which comprises a sequence that hybridizes selectively to a primer used in primer extension of the oligonucleotide, a coding region 612 encoding a peptide or complementary to a region encoding a peptide, and a second primer region 610 for attachment of a universal untranslated region 618 and/or a capture agent 616 comprising a capture agent that specifically binds to the affinity tag encoded by region 614. Extension of the single-stranded molecule from primer region 614 and attachment of the untranslated region in step 611 produces a double-stranded template 602 that includes the capture agent positioned on the construct distal to the substrate surface 608. In this example, the encoded region 612 encodes the peptide of interest, and the primer extension region 614 encodes an affinity tag that binds to a capture agent 616, and a stop codon. *In vitro* transcription and translation will produce the fusion peptide 650, comprising both the peptide of interest 620 and the affinity region 622, the latter of which associates with the peptide association moiety 616 following peptide synthesis.

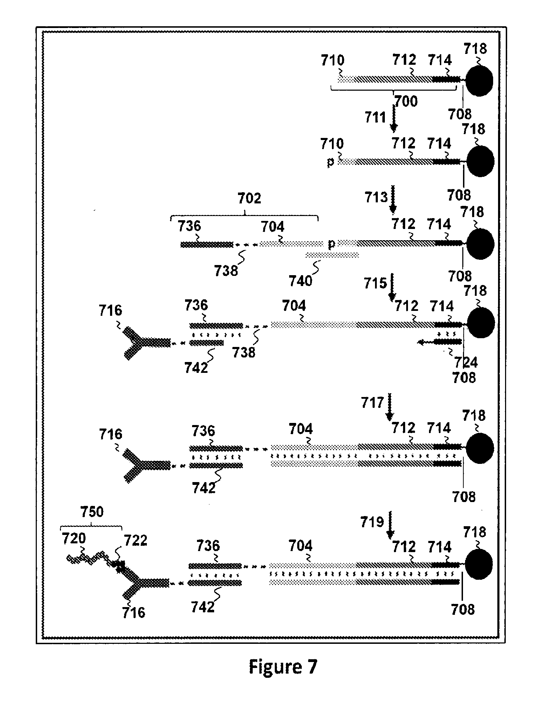

In certain production methods, the oligonucleotides are tethered to different surfaces, *e.g.,* beads provided in solution. FIG. 7 illustrates a more detailed method for constructing an array of the invention using attachment of oligonucleotides to separate surfaces, *e.g.,* beads. The arrays are constructed using oligonucleotide templates 700 comprising a region 710 for introduction of the capture agent, a region 712 encoding the polypeptide of interest, and a region 714 encoding an affinity tag followed by a stop codon. The region 714 also comprises a primer site for primer extension of the single-stranded oligonucleotides, and it is optionally attached to the surface of the bead 718 by a linker molecule 708.

The oligonucleotides 700 on the beads 718 are provided in phosphorylated form, or alternatively phosphorylated in step 711 to allow addition of an oligonucleotide extension. The oligonucleotide extension 702 is then ligated in step 713 to the template oligonucleotide 700 via a splint ligation technique using a ligation oligonucleotide 840 that selectively binds to both the oligonucleotide 800 and the oligonucleotide extension 802. The oligonucleotide extension 702 comprises: a region 704 comprising an untranslated region and a primer region for use in splint ligation; optionally a linker molecule 738; and an oligonucleotide region 736 for attachment of the capture group 716. The next step in production of the array is a combined step 715 to add the capture agent 716 and the primer 724 for primer extension. A capture agent 716 associated with an oligonucleotide 742 that is complementary to region 736 of the oligonucleotide extension 702, and an oligonucleotide 724 to serve as a primer for primer extension are then hybridized to the oligonucleotides on the beads.

A primer extension step 717 is carried out to convert the single-stranded oligonucleotide encoding the peptide of interest and the affinity tag into a double-stranded template for use in *in vitro* transcription and translation. *In vitro* transcription and translation is carried out in step 719 from this double-stranded template, creating a fusion peptide 750 comprising the peptide of interest 720 and an affinity tag 722. The produced fusion peptide 750 is captured by the capture agent 716 as it diffuses from the oligonucleotide template region following translation.

The constructs that are synthesized directly on the beads may be used as synthesized, *i.e.,* as an array of constructs on a collection of separate surfaces, *e.g.,* beads, or they may be arrayed on a planar surface either before or after the synthesis of the oligonucleotide-peptide fusions on their surfaces. In the case where synthesis is carried out before the beads are arrayed, measures are generally taken to prevent interaction of peptides with neighboring constructs, e.g., transcription and translation can be carried out with each bead type bearing a different sequence separated in different wells in a plate to ensure that peptides from a bead et comprising one construct are not captured by a different bead set. These surfaces, e.g., beads can then be isolated and arrayed using technology such as that described in, for example, U.S. Pat No. 7,060,431 issued June 2006 to Chee et al.

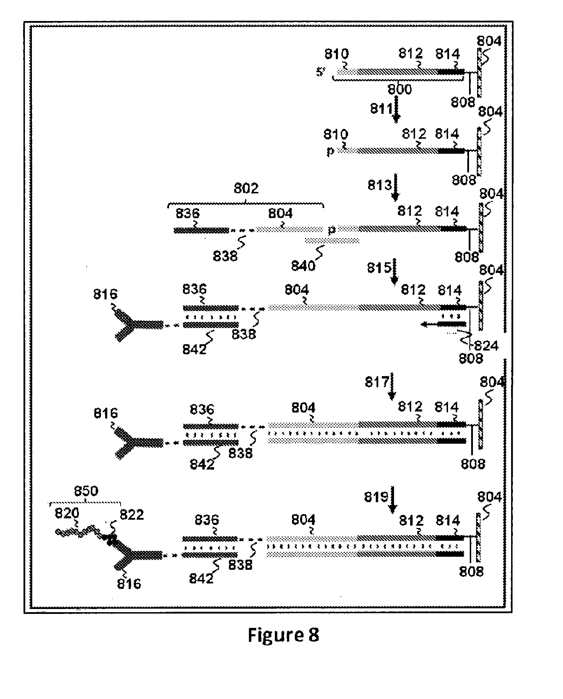

FIG. 8 illustrates a more detailed, preferred method for constructing an array of the invention using attachment of oligonucleotides to a planar array. The arrays are constructed using oligonucleotide templates 800 comprising a region 810 for introduction of the capture agent, a region 812 encoding the polypeptide of interest, and a region 814 encoding an affinity tag followed by a stop codon. The region 814 also comprises a primer site for primer extension of the single-stranded oligonucleotides, and it is optionally attached to the surface of the substrate 804 by a linker molecule 808.

The oligonucleotides 800 on the planar arrays 804 are phosphorylated in step 811 to allow addition of an oligonucleotide extension. The oligonucleotide extension 802 is then ligated in step 813 to the template oligonucleotide 800 via a splint ligation technique using a ligation oligonucleotide 840 that selectively binds to both the oligonucleotide 800 and the oligonucleotide extension 802. The oligonucleotide extension 802 comprises: a region 804 comprising an untranslated region and a primer region for use in splint ligation; optionally a linker molecule 838; and an oligonucleotide region 836 for attachment of the capture group 816. The next step in production of the array is a step 815 to add the capture agent 816 and the primer 824 for use in primer extension. A capture agent 816 conjugated with an oligonucleotide 842 that is complementary to a region 836 of the oligonucleotide extension 802, and the primer 824 can be simultaneously or sequentially hybridized to the oligonucleotides on the slides.

A primer extension step 817 is carried out to convert the single-stranded oligonucleotide encoding the peptide of interest and the affinity tag into a double-stranded template for use in *in vitro* transcription and translation. *In vitro* transcription and translation reactions in step 819 from this double-stranded template create a fusion peptide 850 comprising the peptide of interest 820 and an affinity tag 822. The produced fusion peptide 850 is captured by the capture agent 816 as it diffuses from the oligonucleotide template region and away from the planar surface following translation.

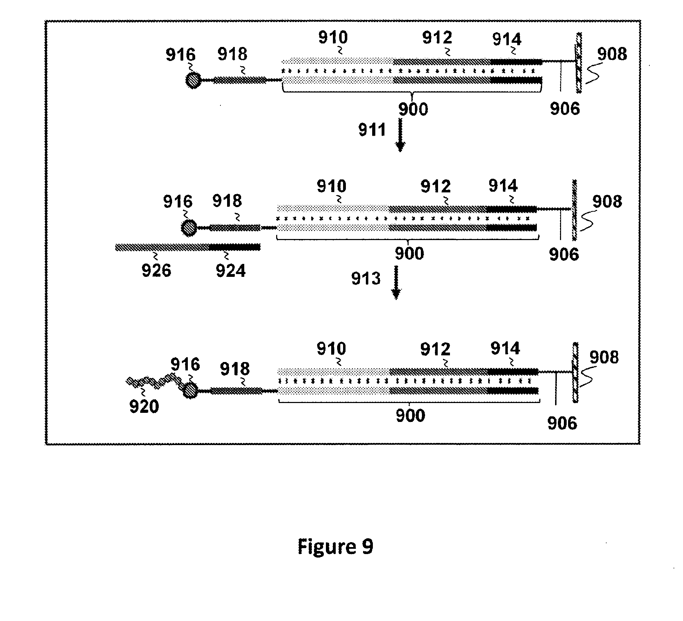

In another example, the constructs of the invention are produced using a puromycin capture method. FIG. 9 illustrates a general method for the construction of such arrays on a planar surface, although it is also envisioned that the arrays could be formed on beads, such as described in FIG. 7. The method begins with double-stranded oligonucleotides 900 arrayed on a substrate surface 908, attached to the surface at either the 3' end or the 5' end of the oligonucleotide, optionally via a linker sequence 906. The oligonucleotides 900 each comprise a coding region 912 encoding a peptide or of interest, a region comprising an RNA affinity tag followed by a stop codon 914, and an untranslated region 910 comprising a transcription promoter, to which an RNA binding region of DNA 918 and a puromycin 916 are attached. An *in vitro* transcription step 911 produces an mRNA molecule comprising a region 926 encoding the peptide of interest and an affinity tag 924. The affinity tag 924 selectively hybridizes to region 918 of the oligonucleotide, tethering the mRNA molecule to the construct. During translation, the ribosome will stall when it reaches the RNA-DNA hybridized region. Following an *in vitro* translation step 913, the peptide 920 is captured by the puromycin residue region 916.

**Peptide Capture through Post-translational Modification**

In specific aspects, a chemically reactive species (e.g., an aldehyde tag) may be added to aid in the construction of the constructs and introduction of labeling elements of other binding regions. For example, introduction of a sulfatase consensus sequence recognized by the formylglycine-generating enzyme results in the site-specific introduction of aldehyde groups into recombinant proteins. This consensus sequence can be between 6-13 amino acids, and the smallest such "aldehyde tags" are no larger than a $His_6$ tag. Enzymatic modification at a sulfatase motif by formylglycine generating enzyme (FGE) generates a formylglycine (FGly) residue, which allows site-specific attachment of a capture agent or other moiety of interest to the peptide by covalent capture on hydrazine or oxime labeled oligo templates. This modification is also reversible, and thus the introduction of this tag into recombinant peptides allows aldehyde-tagged proteins to be reversibly modified with multiple epitopes. Examples of aldehyde tags for use in the present invention are described in, *e.g.,* US2008/0187956; T. Dierks and M.-A. Frese, ChemBioChem 10, 425 - 427 (2009); JS Rush and CR Bertozzi J. Am. Chem. Soc. 9 Vol. 130:37, (2008); J Landgrebe et al., Gene 316 47-56 (2003); I Carrico, Nat Chem. Biology 3:6 (2007); Carlson et al., J Biol Chem. 2008 Jul 18;283(29):20117-25; and Wu et al., Proc Natl Acad Sci U S A. 2009 Mar 3;106(9):3000-5.

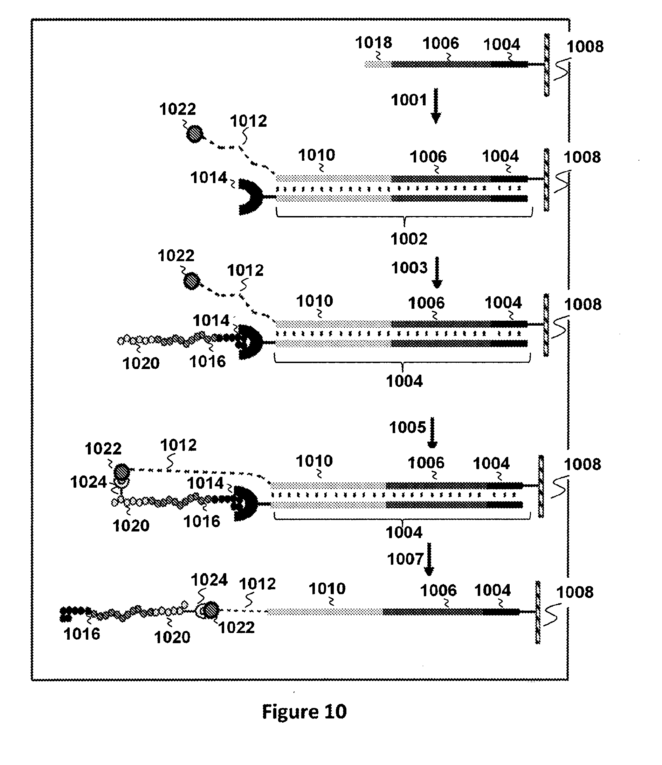

In a more specific approach, the generation of covalently linked peptide arrays is shown in Fig. 10. This approach is an exemplary method for carrying out the more general scheme outlined in Fig. 1, but uses covalent attachment. It utilizes a method for the site-specific introduction of aldehyde groups into recombinant proteins using the 6-mer peptide consensus sequence (LCTPSP, termed as the aldehyde tag) recognized by the formylglycine-generating enzyme (FGE) which oxidizes a cysteine residue to formylglycine was recently reported. In this aspect, an aldehyde tag is to add FGE to the *in vitro* transcription/translation process during peptide array production. The peptides can be made with a C-terminal aldehyde tag, and the cysteine residue in the tag converted to the formylglycine residue that will react with an active group incorporated into the DNA templates on the array surface. This will result in an array of peptides that are covalently attached to their DNA templates.

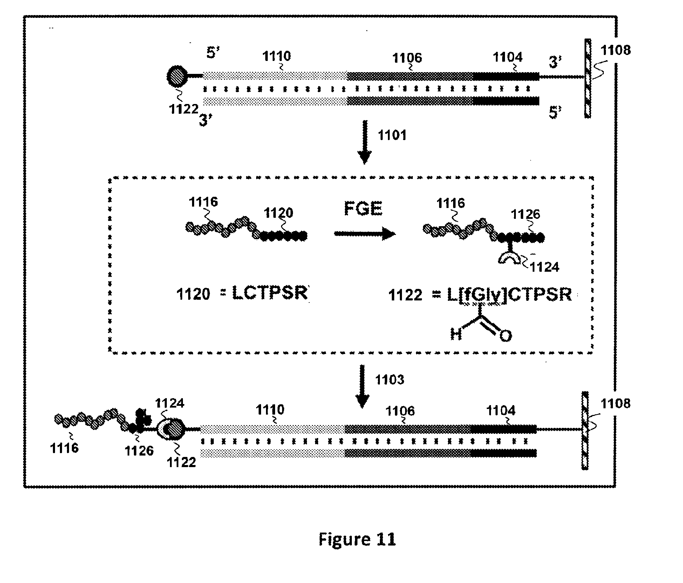

This aspect is illustrated in more detail in FIGs. 10 and 11. Oligonucleotide templates encode the peptide sequences of interest 1006 and a region 1004 comprising an aldehyde affinity tag (LCTPSP) and a stop codon. A universal sequence 1010 containing an untranslated region (UTR) with the T7 promoter and a ribosomal binding site (RBS) is added 1001 to the template via a distal tag 1018 and is used to extend the template to create a double-stranded nucleic acid 1002 comprising a capture group 1014 for the peptide of interest 1016 encoded by 1006 on the strand associated with the substrate 1008. A reactive group 1022 is associated with the other strand of the double-stranded oligonucleotide template 1002, optionally on a linker molecule 1012. Once the peptide comprising the peptide sequence of interest 1016 and the aldehyde tag 1020 is produced 1003, it is captured by interaction of the peptide of interest and the capture group 1014. The aldehyde tag 1020 is converted 1005 to an aldehyde group 1024, and this interacts with reactive group 1022. Upon dissociation 1007 of the strand of the nucleic acid not associated with the substrate 1008, a single-stranded oligonucleotide encoding the peptide of interest 1016 remains associated to the peptide 1016 via the reactive group and the aldehyde group. Optionally, the capture agent 1014 can be removed.

In FIG. 11, peptides with sequences of interest 1116 are made via *in vitro* transcription/translation in the presence of FGE enzyme. As illustrated in detail in FIG. 11, the aldehyde tag 1120 fused to the peptide of interest 1116 can be generated through interaction of a hydrazine or aminooxyacetic acid residue in 1116 with FGE, which converts 1101 the LCTPSR region incorporated in peptides to an aldehyde group 1124. This aldehyde group is used to interact with the reactive group 1122 to associated the peptide of interest 1116 to the oligonucleotide encoding the peptide of interest 1106, the universal sequence and the sequence encoding the aldehyde tag 1104.

**Assay Systems using Sequencing Flow Cells**

In a specific aspect, the coding sequences used for the production of the peptide arrays are provided on the surface of a flow cell, *e.g.,* a flow cell used for sequencing techniques. The coding sequences can be randomly arrayed on the surface of the flow cell. The constructs of the arrays can be created by randomization of bases at defined positions during synthesis of an oligo template, by combining shorter oligos to form a longer template, or by deriving a library of sequences from a genomic DNA or cDNA library. Once the sequences have been placed on the surface of the flow cell, the identity of each sequence can be determined either before or after the production of the tethered protein and its use in a screening assay.

The coding sequences can optionally be clonally amplified *in situ,* with each sequence forming a 'cluster' of clonally amplified DNA molecules, prior to production of the peptide array on the surface. In this specific aspect, very high packing densities can be achieved, e.g., several hundred million clusters can be arrayed on the flow cell surface. The ability to array many millions of DNA templates and determine their sequence opens up possibilities of making large combinatorial protein libraries quite efficiently and inexpensively.

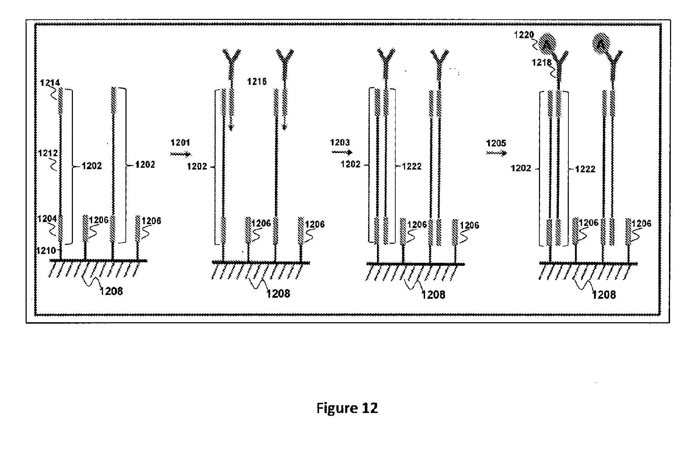

The general scheme using a sequencing flow cell for the array substrate is shown in FIG. 12. Oligonucleotides 1202 comprising the sequence of interest 1212, a linker region 1204 and a primer binding region 1214 are constructed on the flow cell surface 1208. Other linker regions on the surface 1206 are optionally present for the purposes of in situ amplification of the oligonucleotide template. Following sequencing of these constructs, an oligonucleotide-antibody conjugate 1216 is annealed 1201 to the 3'-end of clustered DNA sequences. All DNA sequences code for peptides or proteins and have T7 promoter (or similar) in order to enable efficient transcription and an untranslated region (UTR) required for efficient translation. The oligonucleotide portion of the conjugate 1216 is extended 1203 with DNA polymerase to create an oligonucleotide 1222 complementary to the attached oligonucleotide 1202. A coupled transcription/translation reaction (TNT) 1205 leads to production of peptides or proteins 1220 encoded by clustered DNAs 1202 and their attachment to their own templates. All peptides have C-terminal affinity tag that is used for antibody capturing (not shown). Other capture molecule and affinity tag combinations can be used. Examples of such binding pairs include, but are not limited to, streptavidin and biotin, Ni-NTA and His6 tag, or two chemically reactive groups (aldehyde tag and hydrazine residue).

This approach is also applicable to other formats that permit *in vitro* cloning of single molecules. For example, instead of capture and amplification on the surface of a flow cell using surface PCR, a template molecule can be captured on a bead and amplified by emulsion PCR. This process is used to generate clonal templates for certain next generation sequencing techniques. Although amplification of the individual templates is preferred, in certain aspects single molecule sequences can be determined without the use of clonal amplification.

Optionally, the surface of the flow cell may comprise other linker regions on the surface that are present for the purposes of in situ amplification of the oligonucleotide template. These constructs are sequenced either before construction of the peptide-nucleic acid hybrid or following construction and/or use (not shown). In the former case, the constructs are sequenced prior to production of these peptide-nucleic acid constructs, and an oligonucleotide-antibody conjugates are annealed to the 3'-end of clustered DNA sequences on the surface. Alternatively, the sequence can be determined following construction and/or identification of particular sequences in an assay format.

The antibodies can be loaded on the surface of the flow cell, coupled to the primer portion and directed only to the DNA clusters on the flow cell since the DNA-antibody complex will only hybridize to extended DNAs. Alternatively, the antibodies coupled to oligonucleotides which are complementary to the oligos on the surface can introduced to the flow cell surface. In this aspect, antibodies are loaded over entire surface of the flow cell since the DNA-antibody complexes will hybridize to oligonucleotide primers that are equally distributed over the surface of the flow cell. Peptide arrays are then formed from these DNA templates using one of the described methods herein.

Sequencing technology such as that provided by Illumina™ Genome Analyzer™ technology permits a DNA template to be sequenced from both ends in order to generate a 'paired end' pair of sequence reads. In the case of the Illumina technology, this involves: a) obtaining the sequence of one end of the pair by sequencing one strand; then b) obtaining the sequence of the other end of the pair by sequencing the reverse complement strand. Processes are carried out in order to generate and sequentially present the appropriate strand for sequencing. Therefore, arrays can also be constructed using the second (i.e. reverse complement) strand, simply by choosing the appropriate sequences to conjugate to the capture agent (i.e. in these examples, the antibody).

The arrays of the invention can utilize multiple ligation events to increase the length of the oligonucleotide template and thus the peptide produced through *in vitro* transcription and translation. This can be done through the use of restriction enzyme digestion and ligation, or preferably the use of splint ligation with primers that are complementary to both the oligonucleotide associated with the array and the oligonucleotide that is to be added to the array. For example, a region can be added to all or a subset of the constructs on an array using splint ligation such as that described for the addition of the oligonucleotide comprising the untranslated region. In another example, a pool of oligonucleotides having two or more different sequences can be used with splint ligation primers having corresponding complementary sequences, allowing addition of multiple different variable oligonucleotides to the oligonucleotides associated with the substrate. These approaches could also optionally be combined to create constructs having added regions comprising both constant and variable regions.

These techniques may be especially useful in the creation of an array comprising longer proteins for interrogation. For example, many proteins have various alternatively spliced isoforms that vary only in the domains at the C-terminus or N-terminus, and the arrays of the invention can have the variable regions of these proteins synthesized directly on the substrate surface and the common domains added to these oligonucleotides via ligation. In a specific example, there are at least 27 alternatively-spliced Neural Cell Adhesion Molecule (NCAM) mRNAs produced, and the three main isoforms of NCAM vary only in their cytoplasmic domain. The ability to create longer oligonucleotide templates on the arrays of the invention can provide tools to better elucidate the activity and protein interaction of the various forms of proteins, and give insight into regulation, e.g., for therapeutic development. Numerous other such uses of constructed regions having certain constant domains, e.g., the N-terminus, the C-terminus, active binding sites, enzymatic active regions, etc. are envisioned with the arrays of the invention, as will become clear to one skilled in the art upon reading the present disclosure.

Due to the stable nature of these constructs, in certain assay systems the arrays can also be utilized two or more times. The binding of the peptide to the oligonucleotide via the affinity tag-capture agent interaction is a much tighter bond than that seen in most transient protein-protein interactions, and thus many proteins and compounds used to interrogate the arrays can be effectively removed to allow the array to be used in other interrogations. In addition, the array can be regenerated by removal of all hybridized portions of the constructs, effectively renewing the initial array used for construction of the peptide array. The single-stranded oligonucleotides can be again converted to a double-stranded oligonucleotide, and the remaining steps of the methods of the invention used to recreate a peptide comprising array of the invention.

**Primer Extension**

In various aspects, the initial oligonucleotide is a single-stranded oligonucleotide that is synthesized on the substrate surface and converted to a double-stranded molecule through, e.g., primer extension. Primer extension can be initiated by hybridization of a primer to a primer-binding region on the single-stranded oligonucleotide template where the result after extension is a double-stranded template including the untranslated region, the peptide coding region and the region encoding the affinity tag. Preferably, the primer-binding region incorporates the affinity tag sequence and/or the stop codon sequence, although the primer-binding region may also be located 3' to the affinity tag sequence and the stop codon.

The primer extension process typically utilizes a one or more primers complementary to the oligonucleotide template, which drives synthesis of the opposite strand in the presence of a polymerase and free dNTPs. In such aspects, there is typically a polymerase stop feature introduced into the DNA molecule following the extension of the oligonucleotide template region. This feature may include a synthetic linker, such as a polyethylene glycol (PEG) feature, a nucleotide analog that cannot be used by the polymerase, a nick introduced onto a double-stranded primer region upstream of the template region (e.g., by a nickase or the degradation of uracil) and the like. Multiple primers/ affinity tag encoding regions may be used in a single construct, which may allow a single oligonucleotide to be used with various affinity tag-capture agent binding pairs in construction of the array. The constructs may also have such sequences distal from the substrate surface to aid in selective ligation of capture agents.

**Substrates for Use in the Invention**

Typically, substrates of the invention are nonporous, particularly when random arrays of single molecules are analyzed by hybridization reactions requiring small volumes. Suitable substrates include substrates composed of materials such as glass, polyacrylamide-coated glass, ceramics, silica, silicon, quartz, various plastics, and the like. In one aspect, the substrate is a bead. In another aspect, the substrate is a planar surface. Typically, for conventional uses, the planar surface is in the range of from 0.02 to 20 cm$^2$ or even larger. The limit on substrate size is based on the detection methods used and the ability to resolve (*e.g.,* in the case of fluorescent markers, the ability to optically resolve) the different constructs and/or regions of constructs on the surface. As detection methods continue to improve, substrate size may increase and/or the density of the constructs on a substrate may increase.

The format of the substrates of the present invention includes substantially planar surfaces as well as substrates with introduced variations to the substrate surface, e.g., depressions, wells, pedestals and the like. Such substrates are generally comprised of a material or group of materials having a rigid or semirigid surface or surfaces. In certain aspects, it is desirable to physically separate regions on an array with, for example, wells, raised regions, pedestals, etched holes, or the like. Such substrates can be produced, e.g., using multi-layer coating technologies or other well known techniques in the art. Examples of techniques for production of pattered arrays includes thermal and/or electron beam vapor deposition, replication, transfer, or film deposition; the CVD-type processes (LPCVD, PECVD etc.); PVD-type processes such as sputtering, e.g., DC magnetron sputtering; ion-assisted deposition processes and sol-gel processes. Layers of substrate are optionally transferred onto the base by bonding or molecular adhesion.

In different aspects of the invention, linkers may be used to attach the array constructs to a surface. Numerous types of linkers can be used, and the linker will generally be selected based on the type of construct, (amino acid, nucleic acid, etc.), the desired properties of the linker (length, flexibility) and other similar characteristics. Such linkers may comprise nucleotides, polypeptides, or a suitable synthetic material. The linker structures are preferably hydrocarbon base polymers which are comprised of biocompatible polymeric materials (e.g., polyethylene glycol). Also, the choice of linker depends upon whether the capture agent is associated with the construct at the linker portion of the construct.

In certain aspects, the surface-immobilized constructs comprise a cleavable linker directly attached to the substrate that allows specific constructs to be separated from the substrate. In some aspects, the cleavable linker will be the same or identical for all of the surface-immobilized constructs. In other aspects, certain subsets of constructs on the substrate will have the same cleavable linker, where this cleavable linker differs from the cleavable linkers used with the other subsets on the same substrate surface. This allows certain constructs to be separated from the substrate when others are not.

In certain aspects, a microfluidic gasketing system is used on the substrate surface to effectively isolate each feature on the peptide array to enable control of diffusion during synthesis of the array and analysis using the array. This allows the reaction conditions to be better controlled in one or more portions of the array. For example, the *in vitro* transcription and translation can be carried out for a longer period because the containment system limits diffusion. Also, once peptides are captured, washing and/or reagent exchange steps can be carried out, enabling new reactions to take place, such as covalent linkage of the peptide. Finally, the containment system provided by gasketing allows peptides to be captured on a second surface. In this way, multiple peptide arrays can be produced from an initial template DNA array without the requirement of diffusion through a membrane, such as required in He M Nat. Methods 5:175-177 (2008).

**Binding Pairs for Peptide Capture**

Numerous binding pairs can be used to design the affinity tags and capture agents used in the arrays of the invention. These include, but are not limited to, streptavidin and short streptavidin binding peptides such as StrepTag *(Schmidt et al.,* 1996; Schmidt & Skerra, 1994; Skerra & Schmidt, 2000), StrepTag II (Schmidt & Skerra, 2007; Voss & Skerra, 1997), and HPQ motifs (Giebel *et al.,* 1995; *Helms et al.,* 2007); oligo histidine peptide tags and His6 binding groups (Crowe *et al.,* 1994; Smith *et al.,* 1988); FLAG peptide tags and His6 peptide group; biotin and streptavidin, biotin and avidin, and antibody-antigen pair, and the like.

The strength of the interaction of a peptide binding pair can be characterized by its "binding affinity" of one part of the binding pair to a given binding site or epitope on the other member of the binding pair. For example, in the field of immunology, antibodies are characterized by their "binding affinity" to a given binding site or epitope. Every antibody is comprised of a particular 3-dimensional structure of amino acids, which binds to another structure referred to as an epitope or antigen.

The selective binding of a binding partner to a composition is a simple bimolecular, reversible reaction, not unlike the binding of an antibody to its antigen. For example, if the antibody is represented by Ab and the antigen by Ag, the reaction can be analyzed by standard kinetic theory. Assuming a single binding site the reaction is represented by the equation I as follows:

$$\text{I.} \qquad Ag + Ab \underset{k_2}{\overset{k_1}{\rightleftharpoons}} Ag\text{-}Ab$$

where Ag-Ab is the bound complex. The forward and reverse binding reactions are represented by rate constants $k_1$ and $k_2$ respectively. The "binding affinity" of the antibody to the antigen is measured by the ratio of complexed to free reactants at equilibrium. The lower the concentration of the reactants at equilibrium, the higher the binding affinity of the antibody for the antigen. In the field of immunology, the binding affinity is represented by an "affinity constant" which is represented by the symbol "$K$" or sometimes referred to as "$K_a$". The "$K$" is defined by the equation II as follows:

$$\text{II.} \qquad K = \frac{[Ag - Ab]}{[Ag][Ab]} = \frac{k_1}{k_2}$$

where the brackets denote concentration in moles per liter or liters per mole.

A typical value for the binding affinity $K_a$ which is also referred to as "K" and is the "affinity constant" which for a typical antibody is in a range of from about

$10^5$ to about $10^{11}$ liters per mole. The $K_a$ is the concentration of free antigen needed to fill half the binding sites of the antibody present in solution with the antigen. If measured in liters per mole a higher $K_a$ (*e.g.* $10^{11}$) or higher affinity constant indicates a large volume of solvent, a very dilute concentration of free antigen, and as such indicates the antibody has a high binding affinity for the epitope.

If the $K_a$ is measured in moles per liter a low $K_a$ (*e.g.* $10e^5$) indicates a less concentrated solution of the free antigen needed to occupy half of the antibody binding sites, and as such a high binding affinity.

Equilibrium is achieved in order to measure the $K_a$. More specifically, the $K_a$ is measured when the concentration of antibody bound to antigen [Ag-Ab] is equal to the concentration of the antibody [Ab]. Thus, [Ag-Ab] divided by [Ab] is equal to one. Knowing this, the equation II above can be resolved to the equation III as follows:

$$\text{III.} \qquad K = \frac{1}{[Ag]}$$

In equation III the units for $K$ are liters per mole. Typical values in liters per mole are in a range of from about $10^5$ to about $10^{11}$ liters per mole.

The inverse of the above equation is K = [Ag] where the units for $K$ are in moles per liter, and the typical values are in a range of $10^{-6}$ to $10^{-12}$ moles per liter. The above shows that typical binding affinities can vary over six orders of magnitude. Thus, what might be considered a useful antibody might have 100,000 times greater binding affinity as compared to the binding affinity of what might be considered a different antibody, which is also considered useful.

## Resolvability of Individual Constructs on the Array

Multiple approaches to array construction can be used to ensure that the individual constructs on the array are resolvable when used in assays with various interrogation techniques, even when the constructs are at a very high density on the array. Since there is a certain amount of diffusion of the peptide produced from an individual construct, various configurations of constructs on an array can be used to ensure that this diffusion does not hamper the ability to identify binding to a particular construct, and in fact in certain aspects the diffusion can be used as a beneficial feature.

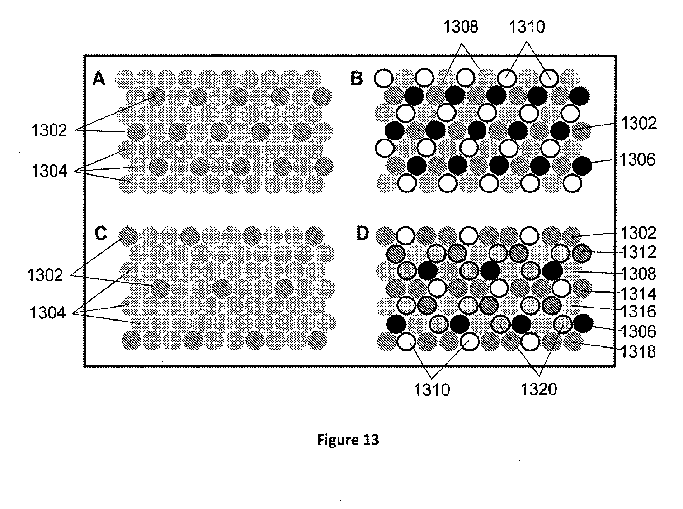

In certain aspects of the invention, the resolvability of the constructs is enhanced by providing one or more features on the array that are "empty", or without a construct or capture agent, providing additional space that prevents excessive diffusion of a peptide from its construct to neighboring constructs. Peptide diffusion following *in vitro* translation is more easily controllable over relatively short distances (*e.g.*, 75-225 $\mu$m), and having empty features between constructs, and preferably between 0 to 2 empty features, allows the resolvable and reproducible identification of individual constructs, thus allowing the identification of binding of an agent of interest to a specific peptide on the array. As illustrated in FIG. 13, arrays having individual constructs comprising the same capture agent (FIG. 13A) may have one empty feature 1304 between the coding constructs 1302. When using peptide for which diffusion is projected to be greater, two or more empty features can be used between constructs. FIG. 13C illustrates an array having two empty features 1304 between coding constructs 1302 having the same capture agent (FIG. 13C). In each of these aspects, each of the coding constructs 1302 encode the same affinity tag, which specifically binds to the capture agent.

In another aspect, multiple capture agents and affinity tags can be used for with the individual constructs, and constructs comprising the different capture agent-affinity tag pairs are interspersed on the array in a configuration to prevent diffusion and inadvertent capture of a peptide created from a construct by its neighboring constructs. For example, non-specific signal due to diffusion of approximately 150 $\mu$m or more at a density of 100%, as shown in FIG. 13B, can be prevented by using four sets of constructs (1302, 1306, 1308 and 1310), each set having a different affinity tag - capture agent pair. In another example, non-specific signal due to diffusion of approximately 225 $\mu$m or more, as shown ion FIG. 13D, can be prevented by using nine sets of constructs (1302, 1306, 1308, 1310, 1312, 1314, 1316, 1318, 1320), each set having a different affinity tag - capture agent pair. Even though diffusion will still take place, the diffusing peptides will not be captured at adjacent features because these will have incompatible capture agents.

In addition to aiding with the diffusion problem, use of multiple capture agent-affinity tag pairs enables the use of multiple constructs having the same peptide of interest, each associated with a capture agent targeting a subset of the constructs on the array. This allows the same peptide of interest to be represented on the array with different capture agent-affinity tag pairs. For example, some peptides of interest may be incompatible with certain peptide affinity tags. The use of different capture agent-affinity tag pairs allows the association of a peptide of interest with more than one such pair, which will to mitigate such effects by representing peptides with two different affinity tags rather than just one.

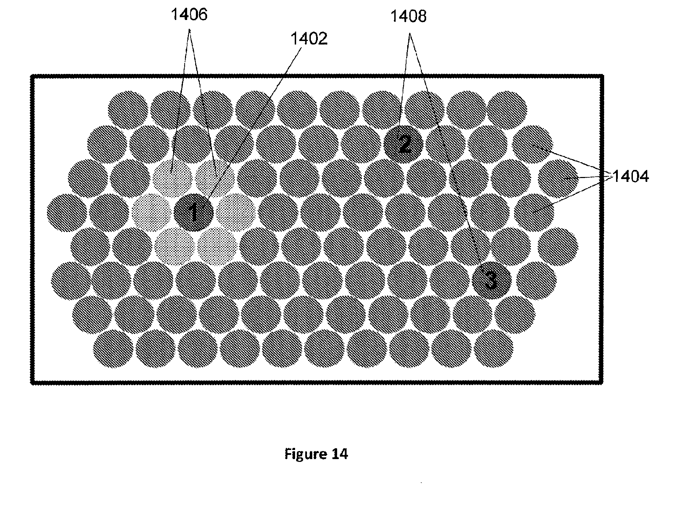

In other aspects, the diffusion of a peptide to its surrounding constructs can be used as an advantage in determining positive results on an array. The small amount of peptide diffusion between constructs having the same capture agent can actually be used to confirm a positive binding result at a particular construct, as the small amount of diffusion to surrounding constructs provide a "halo" effect of binding, and thus an identifiable but lesser signal on the constructs surrounding the one to which an agent selectively binds. FIG 14 illustrates an array having a construct 1402 which selectively binds to an agent and which registers as a positive result in a binding assay. The array also has numerous constructs having no signal 1404 and certain other constructs that spuriously register as false positives. Due to peptide diffusion from the construct 1402 to the surrounding constructs 1406, the surrounding constructs 1406 show a lesser but detectable binding as compared to the true positive construct 1402. The difference in binding between the positive construct 1402 and its neighbors 1406 is statistically significant, thus still allowing identification of the true positive 1402. This halo effect differentiates a binding activity on a construct from other potential spurious sources of a positive result, such as shown in constructs 1408, serving as an internal control mechanism to reduce the number of false positives in an assay.

**EXAMPLES**

The following examples are put forth so as to provide those of ordinary skill in the art with a complete disclosure and description of how to make and use the present invention, and are not intended to limit the scope of what the inventors regard as their invention, nor are they intended to represent or imply that the experiments below are all of or the only experiments performed. The present embodiments are, therefore, to be considered in all respects as illustrative and not restrictive.

Efforts have been made to ensure accuracy with respect to numbers used *(e.g.,* amounts, temperature, etc.) but some experimental errors and deviations should be accounted for. Unless indicated otherwise, parts are parts by weight, molecular weight is weight average molecular weight, temperature is in degrees centigrade, and pressure is at or near atmospheric.

**Example 1: Oligonucleotide Template Design and Synthesis**

Single-stranded oligonucleotides were used for the construction of the arrays. The initial oligonucleotides were 60-mers comprising common regions: a region encoding an affinity tag, either a FLAG peptide (DYKDDDDK)(SEQ ID NO: 1) or its shorter version FLAGS (DDDDK) at the 3'-end; a region encoding a peptide of interest, either an HA peptide (YPYDVPDYA)(SEQ ID NO:2) or an AU1 peptide (DTYRYIDYA)(SEQ ID NO:3); and a primer region for the attachment of a universal untranslated region. The primer region was designed to allow for the addition of a long untranslated region comprising a T7 promoter region at the 5'-end followed by a ribosomal binding site (RBS).

Oligonucleotides were synthesized on an Expedite 8909 DNA synthesizer, using formylindole phosphoramidite (Glen Research, Sterling, VA) to introduce an aldehyde group at either the 5'- or 3'-terminus of the oligonucleotides. In the case of 3'-end modification, the synthesis was started on dT-CPG support resulting in a Formylindol-dT residue at the 3'-end of all oligonucleotides. Internal Cy5 modifier phosphoramidite (*Glen Research*) was used to introduce Cy5 dye at the 3'-end of oligonucleotides. In this case, synthesis was started on dA-CPG support resulting in a Cy5-dA residue at the 3'-end of oligonucleotides.

**Example 2: Production of Arrays on Beads**

A method was developed for synthesizing arrays of the invention on amino-modified silica beads using established protocols. First, amino groups were incorporated into 3 um silica beads (*Bangs Labs*) by treatment with 0.5% 3-aminopropylthriethoxysilane solution in ethanol (*Aldrich*). Next, the beads containing amino groups were treated with 0.1M cyanuric chloride solution (*Aldrich*) containing 0.2M diisopropylethylamine in acetonitrile followed by treatment with 2% hydrazine (*Aldrich*) solution in DMF. Washing with ethanol, acetonitrile and DMF was carried out between each step respectively. This process resulted in beads containing hydrazine triazine groups on their surfaces. The oligonucleotides with 3'-aldehyde groups were covalently attached to the beads. The reaction was performed in 100 mM Na-citrate buffer, pH 5.0, containing 1.5M NaCl overnight at room temperature. After overnight incubation, the beads were washed three times with water, two times with ethanol, resuspended at 10% bead content in ethanol and refrigerated until used.

The oligonucleotides covalently linked to the beads comprise a primer region for introduction of the capture agent, an untranslated region comprising a transcriptional start site and an RBS, a region encoding either the HA or the AU1 peptide of interest, and a region encoding a FLAG affinity tag followed by a stop codon. The FLAG region also comprises a primer site for primer extension of the single-stranded oligonucleotides. In addition, the oligonucleotides comprise a 5' phosphate to allow ligation of the oligonucleotide extensions.

0.05 mg of the prepared silica beads were used in each reaction. For the removal of solutions between the various described incubations, the beads were concentrated as a pellet using a conventional benchtop centrifuge, and the liquid was carefully aspirated so as not to disturb the pellet. The beads were placed in 0.2 ml PCR tubes. 50 $\mu$l of 1XPBST [3mM $NaH_2PO_4$, 1mM $KH_2PO_4$, 150mM NaCl, 0.05% Tween 20 (pH 7.4)] was added and the beads were washed once. The 1XPBST was removed, 50 $\mu$l of KB solution [50 mM Tris-HCl, 10 mM $MgCl_2$ (pH 7.5)] solution was added and the beads were washed once in this buffer.

An oligonucleotide extension comprising an untranslated region and region for use in splint ligation, a PEG linker molecule; and a a T30 oligonucleotide for attachment of the capture group was ligated to the oligonucleotide linked to the bead using a splint primer that hybridizes to both the substrate-linked oligonucleotide and the extension primer.

A ligation mixture comprising 37.5 $\mu$l $H_2O$, 5 $\mu$l

10x T4 DNA Ligase Buffer (NEB), 5 μl of preannealed extension and splint oligonucleotides (10 μM) and 7.5 μl T4 DNA Ligase (400,000 U/ml, NEB) was added to each tube, and incubated for 30 minutes at room temperature in a rotator.

Following the ligation reaction, the beads were treated to denature the splint ligation primer from the ligated oligonucleotide extension and template oligonucleotide linked to the bead. The beads were washed three times with 50 μl 1XPBST. The beads were treated with 50 μl 95% aq Formamide and 1 mM EDTA, incubated five minutes at room temperature, and this treatment was repeated an additional time. The beads were then washed three times with 50 μl 1XPBST.

An anti-FLAG antibody capture agent conjugated with an oligonucleotide that is complementary to a region of the oligonucleotide extension, and an oligonucleotide to serve as a primer for primer extension were then hybridized to the oligonucleotides on the beads. The beads were washed once with 50 μl 1XHyb Buffer [450 mM NaCl, 30 mM sodium dihydrogenphosphate, 13mM EDTA, 0.025% Tween-20 (pH 7.4)]. 50 μl of a capture-agent primer solution [22.5 μl H$_2$O, 25 μl 2X Hyb Buffer, 0.5 μl extension primer (100μM; TTACT-TATCGTCGTCGTC)(SEQ ID NO:4), 2 μl capture agent (A30-Cy5-anti-FLAG -IgG ~ 6.7 μM)] was added to the beads, and incubated at room temperature for 30 minutes in a rotator.

Primer extension was carried out to convert the single-stranded oligonucleotide encoding the peptide of interest and the FLAG affinity tag into a double-stranded, bead linked oligonucleotide. The beads with the primer extension primer hybridized to the bead-linked oligonucleotide were washed twice with 50 μl of 1XHyb Buffer, and once with 50 μl 1XReactII [50mM Tris HCl, 10mM MgCl2, 50mM NaCl, (pH 8)]. 50 μl of an extension mixture [35 μl H$_2$O , 5 μl 10x React II, 5 μl 25mM dNTPs, 5 μl 2 U/μl Diluted DNA Pol I (Large Fragment, (Invitrogen, Carlsbad, CA)] was added and the beads were incubated at 37°C for 45 minutes in a rotator.

*In vitro* transcription and translation were carried out on the double-stranded template to produce a fusion peptide comprising the FLAG affinity tag and the peptide of interest. Following primer extension, the beads were washed with 50 μl 1XReactII and then rinsed twice with 50 μl IVTT buffer [50mM Hepes-KOH , 13mM Mg-Acetate, (pH 7.6)]. PURExpress solution (70 μl H$_2$O, 125 μl Solution A, 50 μl Solution B, , 5 μl 8 U/μl Murine RNase Inhibitor, NEB) was pre-heated at 37°C for 30 minutes, and 12.5 μl TNT solution was then added to the beads, which were incubated for the 60 minutes at 37°C in a rotator.

The peptides of interest were detected on the beads by primary antibodies that recognize an epitope on the respective peptides of interest. The primary antibody was used at a concentration of 67 nM, and the beads were incubated in the presence of the primary antibody for 30 minutes at room temperature. Incubation was performed with a labeled secondary antibody that binds specifically to the primary antibody at a concentration of 17nM. The beads were imaged using a DM6000B automated fluorescence microscope and imaging system.

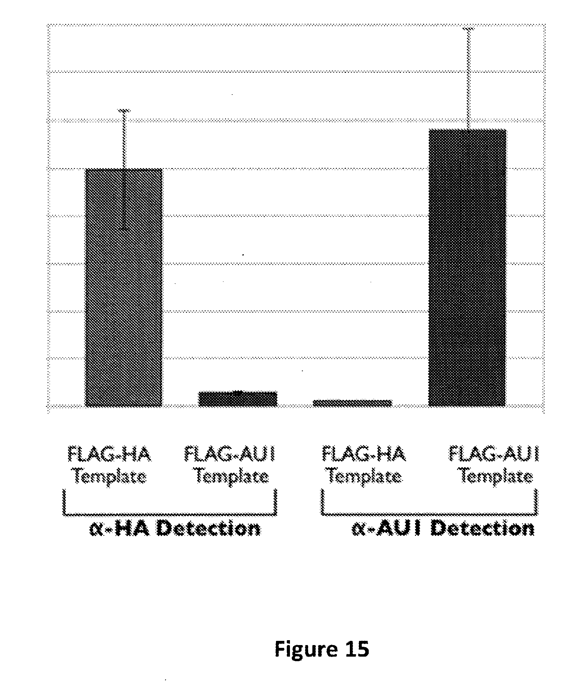

The results of the experiment are as shown in the graph in FIG. 15. The constructs comprising the HA peptide of interest were well detected by the primary anti-HA antibody, which did not detect the AU1 peptide to any significant level. Similarly, the AU1 peptide of interest were well detected by the primary anti-AU1 antibody, which did not detect the HA peptide to any significant level.

**Example 3: Arrays on Glass Slides**

The arrays were constructed using oligonucleotides comprising a primer region for introduction of the capture agent, a region encoding an AU1 (DTYRYIDYA)(SEQ ID NO:5), AU5 (TDFYLKDYA)(SEQ ID NO:6), HA (YPYDVPDYA)(SEQ ID NO:7), or V5 (IPNPLLGLD)(SEQ ID NO:8) 9-mer peptide, and a region encoding a FLAG affinity tag followed by a stop codon. The region also comprised a primer site for hybridization of a primer to be used for primer extension of the single-stranded oligonucleotides.

Glass slides with covalently linked attached oligonucleotides were created from amino modified slides and oligonucleotides with a 5'-aldehyde group. Glass ES microscope slides containing amino groups (*Erie Scientific)* were treated with 0.1M cyanuric chloride solution (*Aldrich*) containing 0.2M diisopropylethylamine in acetonitrile followed by the treatment with 2% hydrazine solution in DMF. Washing with acetonitrile and DMF was carried out between each step. This process resulted in slide surfaces containing hydrazine triazine groups reactive towards aldehyde groups on oligonucleotide. The oligonucleotide templates were covalently attached to the activated slide surfaces via a 3'-aldehyde linkage. To create the slides, 30 μl of 200 μM 5'-aldehyde oligonucleotide 3 in 100 mM Na-citrate buffer, pH 5.1, 1.5M NaCl was placed between two reporter slides. The reaction was allowed to proceed for 18 hours at room temperature in a humidified chamber. The slides were washed three times two times with water, dried and refrigerated until used.

To create the slides, -a submicroliter droplet of 3'-aldehyde modified oligonucleotide (5 μM in citrate buffer [100 mM sodium citrate, 1.5M NaCl, (pH5.0)]) was applied to the slide surface. The reaction was allowed to proceed for 18 hours at room temperature in a humidified chamber. The slides were washed with water, dried and refrigerated until used.

The oligonucleotide slides were then prepared for array construction in a flow through apparatus by the addition of 150 μl of 100% Formamide. 250 μl of 1XPBST was added in two 125 μl aliquots to each slide and incubated for 1 minute and then this wash step was repeated. 250 μl of Blocking solution [1X Hyb Buffer, 5x Denhardt s Solution, and 0.1 mg/ml Denatured Salmon Sperm DNA] was added to each slide in two 125 μl aliquots, and incubated for 5 minutes. 250 μl fresh Blocking solution was added and the slides were incubated for 10 minutes.

450 μl of KB solution [50 mM Tris-HCl (pH 7.5), 10 mM MgCl₂] solution was then added to each slide in 3 equal aliquots, and incubated for 30 seconds at room temperature. This wash step was repeated four times. 250 μl (125 μl, 2 times) of Kinase solution [195 μl H₂O, 25 μl 10x PNK Buffer (NEB, Ipsich, MA), 25 μl 10mM ATP, and 5 μl T4 Polynucleotide Kinase (10u/μl)] was added to each slide, and the slides were then placed into a humidified incubation chamber that had been pre-equilibrated to 37°C.

The oligonucleotide extension comprising an untranslated region and a T30 region for use in splint ligation, a PEG linker molecule, and an oligonucleotide for attachment of the capture group was ligated to the oligonucleotide template using a splint primer, which hybridizes to both the substrate-bound oligonucleotide and the oligonucleotide extension. To prepare the slides for ligation, 450 μl (150 μl, 3 times) of KB solution was then added to the slides in 3 equal aliquots, and the slides were incubated for 30 seconds at room temperature. This wash was repeated two times, and the flow-through chambers comprising the slides were placed in the humidified incubation chamber pre-equilibrated to 4°C in the refrigerator and incubated for 10 minutes.

A ligation mixture comprising 112.5 μl H₂O, 15 μl 10x T4 DNA Ligase Buffer (NEB), 7.5 μl 20U/μl extension oligonucleotides and 2.5 μl T4 DNA Ligase (400,000 U/ml, NEB) was added to each slide, and the slides were incubated for 30 minutes at room temperature. An additional 150 μl aliquot of cold ligation mixture was then added, and the slides were incubated for an additional 30 minutes at room temperature. The resulting structures comprised the oligonucleotide template ligated to the extension oligonucleotide.

Following the ligation reaction, the slides were treated to denature the splint ligation primer from the ligated substrate-linked, extended oligonucleotide. The slides were incubated with 450 μl of KB solution [50 mM Tris-HCl (pH 7.5), 10 mM MgCl₂] added to each slide in 3 equal aliquots, for 30 seconds at room temperature. The slides were then loaded with 450 μl 95% aq. Formamide with 1 mM EDTA, added in 3 equal aliquots, and incubated for 1 minute. This was replaced with another 450 μl 95% aq. Formamide with 1 mM EDTA, added in 3 equal aliquots, and incubated for an additional 5 minutes.

An anti-FLAG antibody capture agent conjugated with an oligonucleotide that is complementary to region of the extension oligonucleotide, and an oligonucleotide to serve as a primer for primer extension were then hybridized to the oligonucleotides on the slides. 450 μl 1XHyb Buffer was introduced to each slide, and incubated for 30 seconds at room temperature. This was repeated four times. A capture-agent-primer solution [67.5 μl H₂O, 75 μl 2X Hyb Buffer, 1.5 μl extension primer (100μM; TTACTTATCGTCGTCGTC)(SEQ ID NO:9), 6 μl capture agent (A30Cy5-anti-FLAG-IgG ~ 6.7 μM)] was added to the flow through chamber, and it was incubated in a humidified chamber at room temperature for 30 minutes.

Following the ligation reaction, the slides were treated to denature the splint ligation primer from the ligated substrate-linked, extended oligonucleotide. The slides were incubated with 450 μl of KB solution [50 mM Tris-HCl (pH 7.5), 10 mM MgCl₂] added to each slide in 3 equal aliquots, for 30 seconds at room temperature. The slides were then loaded with 450 μl 95% aq. Formamide with 1 mM EDTA, added in 3 equal aliquots, and incubated for 1 minute. This was replaced with another 450 μl 95% aq. Formamide with 1 mM EDTA, added in 3 equal aliquots, and incubated for an additional 5 minutes.

An anti-FLAG antibody capture agent conjugated with an oligonucleotide that is complementary to region of the extension oligonucleotide, and an oligonucleotide to serve as a primer for primer extension were then hybridized to the oligonucleotides on the slides. 450 μl 1XHyb Buffer was introduced to each slide, and incubated for 30 seconds at room temperature. This was repeated four times. A capture-agent-primer solution [67.5 μl H₂O, 75 μl 2X Hyb Buffer, 1.5 μl extension primer (100μM; TTACTTATCGTCGTCGTC)(SEQ ID NO:10), 6 μl capture agent (A30Cy5-anti-FLAG-IgG ~ 6.7 μM)] was added to the flow through chamber, and it was incubated in a humidified chamber at room temperature for 30 minutes.

Primer extension was carried out to convert the substrate-linked, single-stranded oligonucleotide encoding the peptide of interest and the FLAG affinity tag into a double-stranded substrate-linked molecules. The slides with the primer extension primer hybridized to the slide-linked oligonucleotides were treated with 450 μl of 1XHyb Buffer, added in three equal aliquots, incubated for 30 seconds, and then twice treated with 450 μl 1XReactII and incubated for 30 seconds. 150 μl of an extension mixture [105 μl H₂O , 15 μl 10x React II, 15 μl 25mM dNTPs, 15 μl 2 U/μl Diluted DNA Pol I (Large Fragment, (Invitrogen, Carlsbad, CA)) was added and the slides were incubated in a humidified incubation chamber pre-equilibrated to 37°C for 45 minutes.

*In vitro* transcription and translation were carried out on the double-stranded substrate-linked product to produce a fusion peptide comprising the FLAG affinity tag and the peptide of interest, either HA or AU1. Following primer extension, 450 μl 1XReactII was added in 3 equal aliquots, and incubated for 30 seconds at room temperature The slides were then rinsed twice with 450 μl IVTT buffer for 30 seconds each rinse.

PURExpress solution (70 μl H₂O, 125 μl Solution A, 50 μl Solution B, 5 μl 8 U/μl Murine RNase Inhibitor, NEB) was pre-heated at 37°C for 30 minutes. The flow-through chambers were placed in a humidified incubation chamber pre-equilibrated to 37°C, and incubated for between 0.5 - 60 minutes at 37°C.

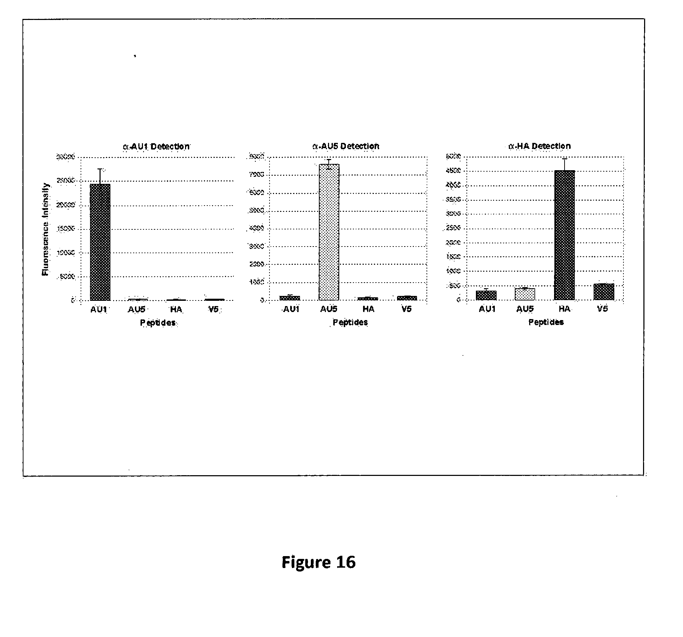

The peptides of interest were detected on the array surface by primary antibodies that recognize an epitope on the peptide of interest. The primary antibody was added at a concentration of 6.7 nM (in Superblock, ThermoSci), and the slide was incubated for 15 minutes at room temperature. This step was then repeated. After three washes with 450 $\mu$l of 1xPBST, incubation was performed with a labeled secondary antibody that binds specifically to the primary antibody at a concentration of 17 nM (in Superblock) for 1.5 min. This step was repeated once. The slide was washed three times with 450 $\mu$l of 1XPBST and then removed from the flow through chamber. After 1 further wash with 1xPBST, the slide was dried and then imaged in a PE ScanArray Lite microarray reader (GSI Luminomics). The bar graph in FIG. 16 sets forth the results of the antibody fluorescence detection on the slides. The AU1, AU5, HA and V5 9-mer peptides were constructed on three microscope slides in the same layout, with three spots of each peptide per slide, and detected specific signal by treating each slides with a specific antibody for the AU1, AU5, or HA peptides. The V5 peptide provided a negative control for all three detection conditions. As shown in the figure, the peptides were selectively detected by the appropriate antibody: three AU1 peptides were detected with the anti-AU1 antibody (16A), three AU5 peptides were detected with the anti-AU5 antibody (16B), and three HA peptides were detected with the anti-HA antibody (16C).

## Claims

1. A method for constructing an array, comprising:

    a) chemically synthesizing a set of oligonucleotides directly on a solid substrate, wherein each oligonucleotide encodes a peptide of interest;
    b) attaching one or more additional oligonucleotides comprising an untranslated region, and a sequence encoding an affinity tag to each oligonucleotide encoding the peptide of interest, to create an extended oligonucleotide on the solid substrate, wherein the extended oligonucleotide comprises a capture agent capable of selectively binding to the affinity tag;
    c) performing *in vitro* transcription and translation utilizing each extended oligonucleotide as a template for production of a peptide comprising the peptide of interest and the affinity tag; and
    d) capturing the peptide produced in step (c) by interaction between the affinity tag and the capture agent of the extended oligonucleotide; thereby attaching the produced peptide on the solid substrate.

2. The method of claim 1, wherein the untranslated region comprises a transcriptional start site and a ribosomal binding site.

3. The method of claim 1, wherein each oligonucleotide in step (a) is associated with the substrate surface via a linker.

4. The method of claim 1, wherein the capture agent is associated with the extended oligonucleotide via a linker.

5. The method of claim 1, wherein the substrate comprises beads.

6. The method of claim 1, wherein the set of oligonucleotides comprises at least 10,000 different oligonucleotides chemically synthesized directly on the substrate.

7. A method of constructing an array comprising the steps of:

    (a) providing a surface comprising two or more oligonucleotide constructs, each oligonucleotide construct comprising a region encoding a peptide of interest;
    (b) extending each oligonucleotide construct by attaching a second oligonucleotide to each oligonucleotide construct, wherein the second oligonucleotide comprises a ribosomal binding site, thereby generating an extended oligonucleotide construct;
    (c) attaching a capture agent to each oligonucleotide construct or each second oligonucleotide, either before or after each second oligonucleotide is attached to each oligonucleotide construct; and
    (d) subjecting each extended oligonucleotide construct to a transcription and translation event to produce a peptide comprising the peptide of interest and an affinity tag wherein the affinity tag is encoded by either the oligonucleotide construct, the second oligonucleotide or both, wherein the peptide is captured by the capture agent following translation thereby forming a construct comprising the peptide associated with the extended oligonucleotide construct encoding the peptide.

8. A method of constructing an array, comprising the steps of:

    (a) providing a surface comprising two or more constructs, each construct comprising a single-stranded oligonucleotide encoding a peptide of interest and an affinity tag, or a single-stranded oligonucleotide having a sequence that is the reverse complement of a sequence encoding a peptide of interest and an affinity tag;

(b) converting the single-stranded oligonucleotide into a double-stranded oligonucleotide;

(c) introducing an untranslated region comprising a ribosomal binding site to the 5' end of the double-stranded oligonucleotide, wherein the untranslated region is associated with a capture agent capable of selectively binding to the affinity tag, thereby generating an extended double-stranded oligonucleotide; and

(d) subjecting the extended double-stranded oligonucleotide to a transcription and translation event to produce a peptide comprising the peptide of interest and the affinity tag;

wherein the peptide produced in step (d) is captured by the capture agent of the construct following translation, thereby forming a construct comprising the peptide associated with the extended double-stranded oligonucleotide encoding the peptide.

9. A method of constructing an array, comprising the steps of:

(a) providing a substrate comprising a surface with two or more constructs associated with the surface, each construct comprising a single-stranded oligonucleotide encoding a peptide of interest and an affinity tag or a single-stranded oligonucleotide having a sequence that is the reverse complement of a sequence encoding a peptide of interest and an affinity tag;

(b) ligating a second oligonucleotide to the single-stranded oligonucleotide of each construct, wherein the second oligonucleotide comprises an untranslated region comprising a ribosomal binding site;

(c) attaching a capture agent to the second oligonucleotide, wherein the capture agent is capable of selectively binding to the affinity tag;

(d) converting the single-stranded oligonucleotide of each construct into double-stranded oligonucleotide regions; and

(e) subjecting the double-stranded oligonucleotide regions to a transcription and translation event to produce a peptide from the double-stranded oligonucleotide regions, wherein the peptide comprises the peptide of interest and the affinity tag;

wherein the peptide produced in step (e) is captured by the capture agent of the construct.

10. The method of claim 7 or claim 9 wherein the second oligonucleotide is a universal oligonucleotide for the array.

11. The method of any one of claims 7, 9 or 10 wherein in step (c), the capture agent is attached to the second oligonucleotide via hybridization to the second oligonucleotide.

12. An array comprising:

a substrate having a surface; and
two or more individual constructs associated with the surface of the substrate, wherein each individual constructs comprises:

(a) an oligonucleotide associated with the substrate surface, the oligonucleotide comprising a region encoding a peptide of interest, a region encoding an affinity tag, and an untranslated region,;
(b) a peptide comprising the peptide of interest and the affinity tag, wherein the peptide is associated with the oligonucleotide; and
(c) a capture agent capable of selectively binding to the affinity tag, wherein the peptide is associated with the oligonucleotide through binding of the affinity tag to the capture agent;

wherein at least two of the constructs comprise regions encoding the same peptide of interest and different affinity tags.

13. A method of identifying an agent that binds to a peptide of interest, comprising:

providing an array of claim 12 comprising one or more peptides of interest; exposing the array to an agent; and
assessing the binding of the agent to the peptide of interest on the array.

14. The array of claim 12, wherein the region encoding the peptide of interest is chemically synthesized directly on the substrate.

**Patentansprüche**

1. Verfahren zur Konstruktion eines Arrays, das Folgendes umfasst:

a) das chemische Synthetisieren einer Gruppe von Oligonucleotiden direkt auf einem festen Substrat, worin jedes Oligonucleotid für ein Peptid von Interesse kodiert;
b) das Binden eines oder mehrerer zusätzlicher Oligonucleotide, die eine untranslatierte Region und eine Sequenz, die für eine Affinitätsmarkierung kodiert, umfassen, an jedes Oligonucleotid, das für das Peptid von Interesse kodiert, um ein erweitertes Oligonucleotid auf dem festen Substrat zu erzeugen, worin das erweiterte Oligonucleotid ein Einfangmittel umfasst, das in der Lage ist, selektiv an die Affinitätsmarkierung zu binden;

c) das Durchführen von In-vitro-Transkription und -Translation unter Verwendung jedes erweiterten Oligonucleotids als Matrize zur Herstellung eines Peptids, das das Peptid von Interesse und die Affinitätsmarkierung umfasst; und

d) das Einfangen des in Schritt (c) hergestellten Peptids durch Wechselwirkung zwischen der Affinitätsmarkierung und dem Einfangmittel des erweiterten Oligonucleotids; wodurch das hergestellte Peptid an das feste Substrat gebunden wird.

2. Verfahren nach Anspruch 1, worin die untranslatierte Region eine Transkriptionsstartstelle und eine Ribosomenbindungsstelle umfasst.

3. Verfahren nach Anspruch 1, worin jedes Oligonucleotid in Schritt (a) über einen Linker mit der Substratoberfläche verbunden wird.

4. Verfahren nach Anspruch 1, worin das Einfangmittel mit dem erweiterten Oligonucleotid über einen Linker verbunden wird.

5. Verfahren nach Anspruch 1, worin das Substrat Kügelchen umfasst.

6. Verfahren nach Anspruch 1, worin die Gruppe von Oligonucleotiden zumindest 10.000 verschiedene Oligonucleotide umfasst, die direkt auf dem Substrat chemisch synthetisiert werden.

7. Verfahren zur Konstruktion eines Arrays, das die folgenden Schritte umfasst:

(a) das Bereitstellen einer Oberfläche, die zwei oder mehrere Oligonucleotidkonstrukte umfasst, wobei jedes Oligonucleotidkonstrukt eine Region umfasst, die für ein Peptid von Interesse kodiert;

(b) das Erweitern jedes Oligonucleotidkonstrukts durch Binden eines zweiten Oligonucleotids an jedes Oligonucleotidkonstrukt, worin das zweite Oligonucleotid eine Ribosomenbindungsstelle umfasst, wodurch ein erweitertes Oligonucleotidkonstrukt erzeugt wird;

(c) das Binden eines Einfangmittels an jedes Oligonucleotidkonstrukt oder jedes zweite Oligonucleotid entweder bevor oder nachdem das jeweilige zweite Oligonucleotid an das jeweilige Oligonucleotidkonstrukt gebunden wurde; und

(d) das Durchführen eines Transkriptions- und Translationsereignisses an jedem erweiterten Oligonucleotidkonstrukt, um ein Peptid hervorzubringen, das das Peptid von Interesse und eine Affinitätsmarkierung umfasst, worin für die Affinitätsmarkierung entweder das Oligonucleotidkonstrukt, das zweite Oligonucleotid oder beide kodieren, worin das Peptid von dem Einfangmittel nach der Translation eingefangen wird, wodurch ein Konstrukt gebildet wird, das das Peptid in Verbindung mit dem erweiterten Oligonucleotidkonstrukt, das für das Peptid kodiert, umfasst.

8. Verfahren zur Konstruktion eines Arrays, das die folgenden Schritte umfasst:

(a) das Bereitstellen einer Oberfläche, die zwei oder mehrere Konstrukte umfasst, wobei jedes Konstrukt ein einzelsträngiges Oligonucleotid, das für ein Peptid von Interesse und eine Affinitätsmarkierung kodiert, oder ein einzelsträngiges Oligonucleotid mit einer Sequenz, die das reverse Komplement einer für ein Peptid von Interesse und eine Affinitätsmarkierung kodierenden Sequenz ist, umfasst;

(b) das Überführen des einzelsträngigen Oligonucleotids in ein doppelsträngiges Oligonucleotid;

(c) das Einführen einer untranslatierten Region, die eine Ribosomenbindungsstelle umfasst, an das 5'-Ende des doppelsträngigen Oligonucleotids, worin die untranslatierte Region mit einem Einfangmittel verbunden wird, das in der Lage ist selektiv an die Affinitätsmarkierung zu binden, wodurch ein erweitertes doppelsträngiges Oligonucleotid erzeugt wird; und

(d) das Durchführen eines Transkriptions- und Translationsereignisses an dem erweiterten doppelsträngigen Oligonucleotid, um ein Peptid zu produzieren, das das Peptid von Interesse und die Affinitätsmarkierung umfasst;

worin das in Schritt (d) produzierte Peptid von dem Einfangmittel des Konstrukts nach der Translation eingefangen wird, wodurch ein Konstrukt gebildet wird, das das Peptid in Verbindung mit dem für das Peptid kodierenden erweiterten doppelsträngigen Oligonucleotid umfasst.

9. Verfahren zur Konstruktion eines Arrays, das die folgenden Schritte umfasst:

(a) das Bereitstellen eines Substrats, das eine Oberfläche mit zwei oder mehreren Konstrukten in Verbindung mit der Oberfläche umfasst, wobei jedes Konstrukt ein einzelsträngiges Oligonucleotid, das für ein Peptid von Interesse und eine Affinitätsmarkierung kodiert, oder ein einzelsträngiges Oligonucleotid mit einer Sequenz, die das reverse Komplement einer für ein Peptid von Interesse und eine Affinitätsmarkierung kodierenden Sequenz ist, umfasst;

(b) das Ligieren eines zweiten Oligonucleotids an das einzelsträngige Oligonucleotid jedes Konstrukts, worin das zweite Oligonucleotid eine untranslatierte Region umfasst, die eine Ribosomenbindungsstelle umfasst;

(c) das Binden eines Einfangmittels an das zweite Oligonucleotid, worin das Einfangmittel in der Lage ist, selektiv an die Affinitätsmarkierung zu binden;

(d) das Überführen des einzelsträngigen Oligonucleotids jedes Konstrukts in doppelsträngige Oligonucleotidregionen; und

(e) das Durchführen eines Transkriptions- und Translationsereignisses an den doppelsträngigen Oligonucleotidregionen, um aus den doppelsträngigen Oligonucleotidregionen ein Peptid zu produzieren, worin das Peptid das Peptid von Interesse und die Affinitätsmarkierung umfasst;

worin das in Schritt (e) produzierte Peptid von dem Einfangmittel des Konstrukts eingefangen wird.

**10.** Verfahren nach Anspruch 7 oder Anspruch 9, worin das zweite Oligonucleotid ein universelles Oligonucleotid für das Array ist.

**11.** Verfahren nach einem der Ansprüche 7, 9 und 10, worin in Schritt (c) das Einfangmittel an das zweite Oligonucleotid durch Hybridisierung an das zweite Oligonucleotid gebunden wird.

**12.** Array, das Folgendes umfasst:

ein Substrat mit einer Oberfläche; und
zwei oder mehrere einzelne Konstrukte in Verbindung mit der Oberfläche des Substrats, worin jedes einzelne Konstrukt Folgendes umfasst:

(a) ein Oligonucleotid in Verbindung mit der Substratoberfläche, wobei das Oligonucleotid eine Region, die für ein Peptid von Interesse kodiert, eine Region, die für eine Affinitätsmarkierung kodiert, und eine untranslatierte Region umfasst;

(b) ein Peptid, das das Peptid von Interesse und die Affinitätsmarkierung umfasst, worin das Peptid mit dem Oligonucleotid verbunden ist; und

(c) ein Einfangmittel, das in der Lage ist, selektiv an die Affinitätsmarkierung zu binden, worin das Peptid durch Bindung der Affinitätsmarkierung an das Einfangmittel mit dem Oligonucleotid verbunden ist;

worin zumindest zwei der Konstrukte Regionen umfassen, die für das gleiche Peptid von Interesse und verschiedene Affinitätsmarkierungen kodieren.

**13.** Verfahren zur Identifizierung eines Mittels, das an ein Peptid von Interesse bindet und Folgendes umfasst:

das Bereitstellen eines Arrays nach Anspruch 12, das ein oder mehrere Peptide von Interesse umfasst;
das Aussetzen des Arrays gegenüber einem Mittel; und
das Bestimmen der Bindung des Mittels an das Peptid von Interesse auf dem Array.

**14.** Array nach Anspruch 12, worin die für das Peptid von Interesse kodierende Region direkt auf dem Substrat chemisch synthetisiert ist.

**Revendications**

**1.** Procédé de construction d'un réseau, comprenant :

a) la synthèse chimique d'un ensemble d'oligonucléotides directement sur un substrat solide, où chaque oligonucléotide code pour un peptide d'intérêt ;
b) l'attachement d'un ou de plusieurs oligonucléotides supplémentaires comprenant une région non traduite, et une séquence codant pour un marqueur d'affinité à chaque oligonucléotide codant pour le peptide d'intérêt, afin de créer un oligonucléotide étendu sur le substrat solide, où l'oligonucléotide étendu comprend un agent de capture capable de se lier sélectivement au marqueur d'affinité ;
c) la réalisation d'une transcription et d'une traduction *in vitro* en utilisant chaque oligonucléotide étendu en tant que matrice pour la production d'un peptide comprenant le peptide d'intérêt et le marqueur d'affinité ; et
d) la capture du peptide produit à l'étape (c) par une interaction entre le marqueur d'affinité et l'agent de capture de l'oligonucléotide étendu ; en attachant ainsi le peptide produit sur le substrat solide.

**2.** Procédé selon la revendication 1, dans lequel la région non traduite comprend un site d'initiation de la transcription et un site de liaison aux ribosomes.

**3.** Procédé selon la revendication 1, dans lequel chaque oligonucléotide de l'étape (a) est associé à la surface du substrat via un segment de liaison.

**4.** Procédé selon la revendication 1, dans lequel l'agent de capture est associé à l'oligonucléotide étendu via un segment de liaison.

5. Procédé selon la revendication 1, dans lequel le substrat comprend des billes.

6. Procédé selon la revendication 1, dans lequel l'ensemble d'oligonucléotides comprend au moins 10 000 oligonucléotides différents qui sont chimiquement synthétisés directement sur le substrat.

7. Procédé de construction d'un réseau, comprenant les étapes qui consistent à :

   (a) mettre à disposition une surface comprenant deux constructions d'oligonucléotide ou plus, chaque construction d'oligonucléotide comprenant une région codant pour un peptide d'intérêt ;
   (b) étendre chaque construction d'oligonucléotide en attachant un second oligonucléotide à chaque construction d'oligonucléotide, où le second oligonucléotide comprend un site de liaison aux ribosomes, en générant ainsi une construction d'oligonucléotide étendu ;
   (c) attacher un agent de capture à chaque construction d'oligonucléotide ou à chaque second oligonucléotide, soit avant soit après que chaque second oligonucléotide est attaché à chaque construction d'oligonucléotide ; et
   (d) soumettre chaque construction d'oligonucléotide étendu à un événement de transcription et de traduction afin de produire un peptide comprenant le peptide d'intérêt et un marqueur d'affinité, où le marqueur d'affinité est codé par la construction d'oligonucléotide, le second oligonucléotide ou les deux, où le peptide est capturé par l'agent de capture suite à la traduction en formant ainsi une construction comprenant le peptide associé à la construction d'oligonucléotide étendu codant pour le peptide.

8. Procédé de construction d'un réseau, comprenant les étapes qui consistent à :

   (a) mettre à disposition une surface comprenant deux constructions ou plus, chaque construction comprenant un oligonucléotide simple brin codant pour un peptide d'intérêt et un marqueur d'affinité, ou un oligonucléotide simple brin ayant une séquence qui est le complément inverse d'une séquence codant pour un peptide d'intérêt et un marqueur d'affinité ;
   (b) convertir l'oligonucléotide simple brin en un oligonucléotide double brin ;
   (c) introduire une région non traduite comprenant un site de liaison aux ribosomes à l'extrémité 5' de l'oligonucléotide double brin, où la région non traduite est associée à un agent de capture capable de se lier sélectivement au marqueur d'affinité, en générant ainsi un oligonucléotide double brin étendu ; et
(d) soumettre l'oligonucléotide double brin étendu à un événement de transcription et de traduction afin de produire un peptide comprenant le peptide d'intérêt et le marqueur d'affinité ;
où le peptide produit à l'étape (d) est capturé par l'agent de capture de la construction suite à la traduction, en formant ainsi une construction comprenant le peptide associé à l'oligonucléotide double brin étendu codant pour le peptide.

9. Procédé de construction d'un réseau, comprenant les étapes qui consistent à :

   (a) mettre à disposition un substrat comprenant une surface avec deux constructions ou plus associées à la surface, chaque construction comprenant un oligonucléotide simple brin codant pour un peptide d'intérêt et un marqueur d'affinité, ou un oligonucléotide simple brin ayant une séquence qui est le complément inverse d'une séquence codant pour un peptide d'intérêt et un marqueur d'affinité ;
   (b) ligaturer un second oligonucléotide à l'oligonucléotide simple brin de chaque construction, où le second oligonucléotide comprend une région non traduite comprenant un site de liaison aux ribosomes ;
   (c) attacher un agent de capture au second oligonucléotide, où l'agent de capture est capable de se lier sélectivement au marqueur d'affinité ;
   (d) convertir l'oligonucléotide simple brin de chaque construction en régions d'oligonucléotide double brin ; et
   (e) soumettre les régions d'oligonucléotide double brin à un événement de transcription et de traduction afin de produire un peptide à partir des régions d'oligonucléotide double brin, où le peptide comprend le peptide d'intérêt et le marqueur d'affinité ;
   où le peptide produit à l'étape (e) est capturé par l'agent de capture de la construction.

10. Procédé selon la revendication 7 ou la revendication 9, dans lequel le second oligonucléotide est un oligonucléotide universel pour le réseau.

11. Procédé selon l'une quelconque des revendications 7, 9 ou 10, dans lequel à l'étape (c), l'agent de capture est attaché au second oligonucléotide via une hybridation au second oligonucléotide.

12. Réseau comprenant :

   un substrat ayant une surface ; et

deux constructions individuelles ou plus associées à la surface du substrat, où chaque construction individuelle comprend :

(a) un oligonucléotide associé à la surface du substrat, l'oligonucléotide comprenant une région codant pour un peptide d'intérêt, une région codant pour un marqueur d'affinité et une région non traduite ;
(b) un peptide comprenant le peptide d'intérêt et le marqueur d'affinité, où le peptide est associé à l'oligonucléotide ; et
(c) un agent de capture capable de se lier sélectivement au marqueur d'affinité, où le peptide est associé à l'oligonucléotide par une liaison du marqueur d'affinité à l'agent de capture ;
où au moins deux des constructions comprennent des régions codant pour le même peptide d'intérêt et différents marqueurs d'affinité.

**13.** Procédé d'identification d'un agent qui se lie à un peptide d'intérêt, comprenant :

la mise à disposition d'un réseau selon la revendication 12 comprenant un ou plusieurs peptides d'intérêt ;
l'exposition du réseau à un agent ; et
l'évaluation de la liaison de l'agent au peptide d'intérêt sur le réseau.

**14.** Réseau selon la revendication 12, dans lequel la région codant pour le peptide d'intérêt est chimiquement synthétisée directement sur le substrat.

Figure 1

**1E**

**1F**

Figure 1 (cont.)

Figure 2

**Figure 3**

**Figure 4**

**Figure 5**

**Figure 6**

Figure 7

Figure 8

**Figure 9**

**Figure 10**

Figure 11

Figure 12

**Figure 13**

Figure 14

**Figure 15**

**Figure 16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03010176 A
- US 20030087232 A
- US 7556919 B, Chenchik
- US 7291471 B, Sampson
- US 6294336 B, Boyce-Jacino
- US 6291183 B, Pirrung
- US 6284497 B, Sabanayagam
- US 6261776 B, Pirrung
- US 6222030 B, Dellinger
- US 6087112 A, Dale
- US 6077674 A, Schliefer and May
- US 5919523 A, Sundberg
- US 5861242 A, Chee
- US 5856174 A, Lipshutz
- US 5837832 A, Chee
- US 5770722 A, Lockhart
- US 5750669 A, Rosch
- US 5843655 A, McGall
- US 5700637 A, Southern
- US 5723320 A, Dehlinger
- US 5695940 A, Drmanac
- US 5631724 A, Stern
- US 5556752 A, Lockhart
- US 5525464 A, Drmanac
- US 5492806 A, Drmanac
- US 5445934 A, Fodor
- US 5436327 A
- US 7060431 B, Chee
- US 20080187956 A

### Non-patent literature cited in the description

- **HENDERSON G ; BRADLEY M.** *Curr Opin Biotechnol.,* 06 August 2007, vol. 18 (4), 326-30
- **TAPIA VE.** *Methods Mol Biol. 2009,* 2009, vol. 570, 3-17
- **SALISBURY CM et al.** *J Am Chern Soc.,* 18 December 2002, vol. 124 (50), 14868-70
- **FRANK R.** *J Immunol Methods,* 01 September 2002, vol. 267 (1), 13-26
- **HE M ; TAUSSIG MJ.** *Nucleic Acids Res,* 2001, vol. 29, e73
- **RANACHANDRAN N et al.** *Science,* 2004, vol. 305, 86-90
- **HEM.** *Nat. Methods,* 2008, vol. 5, 175-177
- **TAO S-C ; ZHU H.** *Nat. Biotech,* 2006, vol. 24, 1253-1254
- **SAMBROOK et al.** Molecular Cloning. Cold Spring Harbor Laboratory, 1989
- **ANDERSON ; YOUNG.** Quantitative Filter Hybridization. *Nucleic Acid Hybridization,* 1985
- **ALLAWI, H. T. ; SANTALUCIA, J., JR.** *Biochemistry,* 1997, vol. 36, 10581-94
- Genome Analysis: A Laboratory Manual Series. 1999, vol. I-IV
- Genetic Variation: A Laboratory Manual. 2007
- PCR Primer: A Laboratory Manual. 2003
- **BOWTELL ; SAMBROOK.** DNA Microarrays: A Molecular Cloning Manual. 2003
- **MOUNT.** Bioinformatics: Sequence and Genome Analysis. 2004
- **SAMBROOK ; RUSSELL.** Condensed Protocols from Molecular Cloning: A Laboratory Manual. 2006
- **SAMBROOK ; RUSSELL.** Molecular Cloning: A Laboratory Manual. Cold Spring Harbor Laboratory Press, 2002
- **STRYER, L.** Biochemistry. W.H. Freeman, 1995
- **GAIT.** Oligonucleotide Synthesis: A Practical Approach. IRL Press, 1984
- **NELSON ; COX.** Lehninger, Principles of Biochemistry. W. H. Freeman Pub, 2000
- **BERG et al.** Biochemistry. W.H. Freeman Pub, 2002
- **T. DIERKS ; M.-A. FRESE.** *ChemBioChem,* 2009, vol. 10, 425-427
- **JS RUSH ; CR BERTOZZI.** *J. Am. Chem. Soc. 9,* 2008, vol. 130, 37
- **J LANDGREBE et al.** *Gene,* 2003, vol. 316, 47-56
- **I CARRICO.** *Nat Chem. Biology,* 2007, vol. 3, 6
- **CARLSON et al.** *J Biol Chem.,* 18 July 2008, vol. 283 (29), 20117-25
- **WU et al.** *Proc Natl Acad Sci U S A.,* 03 March 2009, vol. 106 (9), 3000-5
- **HE M.** *Nat. Methods,* 2008, vol. 5, 175-177